# EUROPEAN PATENT SPECIFICATION

(11) **EP 2 126 066 B1**
(45) Date of publication and mention of the grant of the patent: **01.05.2013**
(21) Application number: 07734802.7
(22) Date of filing: 19.02.2007
(51) Int. Cl.: C12N 9/22

(54) **LAGLIDADG HOMING ENDONUCLEASE VARIANTS HAVING NOVEL SUBSTRATE SPECIFICITY AND USE THEREOF**
LAGLIDADG-HOMING-ENDONUKLEASE-VARIANTEN MIT NEUER SUBSTRATSPEZIFITÄT UND VERWENDUNG DAVON
VARIANTS D'ENDONUCLÉASE HOMING LAGLIDADG À UNE NOUVELLE SPÉCIFICITÉ DE SUBSTRAT ET LEUR UTILISATION

(43) Date of publication of application: 02.12.2009
(73) Proprietor: CELLECTIS, 93230 Romainville (FR)
(72) Inventor: MONTOYA, Guillermo, E-28794 Madrid (ES); BLANCO, Francisco, E-28017 Madrid (ES); PRIETO, Jesus, E-28030 Madrid (ES)
(74) Representative: Thomas, Dean
(86) International application number: PCT/IB2007/001527
(87) International publication number: WO 2008/102198

(56) References cited:
- WO-A-2006/097784
- WO-A-2007/047859
- SMITH JULIANNE ET AL: "A combinatorial approach to create artificial homing endonucleases cleaving chosen sequences" NUCLEIC ACIDS RESEARCH, vol. 34, no. 22, December 2006 (2006-12), XP009091809 ISSN: 0305-1048
- CHEVALIER BRETT ET AL: "Metal-dependent DNA cleavage mechanism of the I-CreI LAGLIDADG homing endonuclease" BIOCHEMISTRY, AMERICAN CHEMICAL SOCIETY. EASTON, PA, US, vol. 43, no. 44, 9 November 2004 (2004-11-09), pages 14015-14026, XP002412602 ISSN: 0006-2960
- CHEVALIER B ET AL: "Flexible DNA Target Site Recognition by Divergent Homing Endonuclease Isoschizomers I-CreI and I-MsoI" JOURNAL OF MOLECULAR BIOLOGY, LONDON, GB, vol. 329, no. 2, 30 May 2003 (2003-05-30), pages 253-269, XP004454255 ISSN: 0022-2836 cited in the application
- PRIETO JESUS ET AL: "The C-terminal loop of the homing endonuclease I-CreI is essential for site recognition, DNA binding and cleavage", NUCLEIC ACIDS RESEARCH, OXFORD UNIVERSITY PRESS, SURREY, GB, vol. 35, no. 10, 22 April 2007 (2007-04-22), pages 3262-3271, XP009108728, ISSN: 0305-1048, DOI: 10.1093/NAR/GKM183

## Description

The invention relates to a method for engineering LAGLIDADG homing endonuclease variants having novel substrate specificity. The invention relates also to a variant obtainable by said method, to a vector encoding said variant, to a cell, an animal or a plant modified by said vector and to the use of said homing endonuclease variant and derived products for genetic engineering, genome therapy and antiviral therapy.

Meganucleases are by definition sequence-specific endonucleases with large (12-45 bp) cleavage sites that can deliver DNA double-strand breaks (DSBs) at specific loci in living cells (Thierry, A. and Dujon B., Nucleic Acids Res., 1992, 20, 5625-5631). Meganucleases have been used to stimulate homologous recombination in the vicinity of their target sequences in cultured cells and plants (Rouet et al., Mol. Cell. Biol., 1994, 14, 8096-8106; Choulika et al., Mol. Cell. Biol., 1995, 15, 1968-1973; Donoho et al., Mol. Cell. Biol, 1998, 18, 4070-4078; Elliott et al., Mol. Cell. Biol., 1998, 18, 93-101; Sargent et al., Mol. Cell. Biol., 1997, 17, 267-277; Puchta et al., Proc. Natl. Acad. Sci. USA, 1996, 93, 5055-5060; Chiurazzi et al., Plant Cell, 1996, 8, 2057-2066), making meganuclease-induced recombination an efficient and robust method for genome engineering.

The use of meganuclease-induced recombination has long been limited by the repertoire of natural meganucleases, and the major limitation of the current technology is the requirement for the prior introduction of a meganuclease cleavage site in the locus of interest. Thus, the engineering of redesigned meganucleases cleaving chosen targets is under intense investigation.

Such proteins could be used to cleave genuine chromosomal sequences and open new perspectives for genome engineering in wide range of applications. For example, meganucleases could be used to knock-out endogenous genes or knock-in exogenous sequences in the chromosome. It can as well be used for the precise *in situ* correction of mutations linked with monogenic diseases and thereby bypass the risk due to the randomly inserted transgenes encountered with current gene therapy approaches (Hacein-Bey-Abina et al., Science, 2003, 302, 415-419).

Recently, Zinc-Finger DNA binding domains of Cys2-His2 type Zinc-Finger Proteins (ZFPs) were fused with the catalytic domain of the *Fok*I endonuclease, to induce recombination in various cell types: mammalian cultured cells including human lymphoid cells, plants and insects (Smith et al., Nucleic Acids Res, 1999, 27, 674-81; Pabo et al., Annu. Rev. Biochem, 2001, 70, 313-40; Porteus, M.H. and Baltimore, D., Science, 2003, 300, 763; Urnov et al., Nature, 2005, 435, 646-651; Bibikova et al., Science, 2003, 300, 764; Durai et al., Nucleic Acids Res., 2005, 33, 5978-5990; Porteus M.H., Mol. Ther., 2006, 13, 438-446). The binding specificity of ZFPs is relatively easy to manipulate, and a repertoire of novel artificial ZFPs, able to bind many (g/a)nn(g/a)nn(g/a)nn sequences is now available (Pabo *et al.,* precited; Segal, D.J. and Barbas, C.F., Curr. Opin. Biotechnol., 2001, 12, 632-637; Isalan et al., Nat. Biotechnol., 2001, 19, 656-660). However, preserving a very narrow specificity is one of the major issues for genome engineering applications, and presently it is unclear whether ZFPs would fulfill the very strict requirements for therapeutic applications. Furthermore, these fusion proteins have demonstrated high toxicity in Drosophila (Bibikova et al., Science, 2003, 300, 764; Bibikova et al., Genetics, 2002, 161, 1169-1175) and mammalian NIHT3 cells (Alwin et al., Mol. Ther., 2005, 12, 610-617; Porteus, M.H. and Baltimore, D., Science, 2003, 300,763; Porteus, M.H. and Carroll, D., Nat. Biotechnol., 2005, 967-973), a genotoxic effect that is probably due to frequent off-site cleavage (Porteus, M.H., Mol. Ther., 2006, 13, 438-446).

In nature, meganucleases are essentially represented by homing endonucleases (HEs), a family of endonucleases encoded by mobile genetic elements, whose function is to initiate DNA double-strand break (DSB)-induced recombination events in a process referred to as homing (Chevalier, B.S. and Stoddard, B.L., Nucleic Acids Res., 2001, 29, 3757-3774; Kostriken et al., Cell; 1983, 35, 167-174; Jacquier, A. and Dujon, B., Cell, 1985, 41, 383-394). Several hundreds of HEs have been identified in bacteria, eukaryotes, and archea (Chevalier, B.S. and Stoddard, B.L., Nucleic Acids Res., 2001, 29, 3757-3774); however the probability of finding a HE cleavage site in a chosen gene is very low.

Given their biological function and their exceptional cleavage properties in terms of efficacy and specificity, HEs provide ideal scaffolds to derive novel endonucleases for genome engineering. Furthermore, in addition to their exquisite specificity, homing endonuclease have shown to be less toxic than ZFPs, probably because of better specificity (Alwin et al., Mol. Ther., 2005, 12, 610-617; Porteus, M.H. and Baltimore, D., Science, 2003, 300, 763; Porteus, M.H. and Carroll, D., Nat. Biotechnol., 2005, 23, 967-973), two features that become essential when engaging into therapeutic applications.

Data have accumulated over the last decade, allowing a relatively good characterization of the LAGLIDADG family, the largest of the four HE families (Chevalier, B.S. and Stoddard, B.L., Nucleic Acids Res., 2001, 29, 3757-74). LAGLIDADG refers to the only sequence actually conserved throughout the family, and is found in one or (more often) two copies in the protein. Proteins with a single motif, such as I-*Cre*I (Wang et al., Nucleic Acids Res., 1997, 25, 3767-3776) form homodimers and cleave palindromic or pseudo-palindromic DNA sequences, whereas the larger, double motif proteins, such as I-*Sce*I (Jacquier, A. and Dujon, B., Cell., 1985, 41, 383-394) or I-*Dmo*I (Dalgaard et al., Proc. Natl. Acad. Sci. USA, 1993, 90, 5417-5417) are monomers and cleave non palindromic targets. Nine different LAGLIDADG proteins have been crystallized with or without bound DNA, showing a very striking core structure conservation that contrasts with the lack of similarity at the primary sequence level (Heath et al., Nature Struct. Biol., 1997, 4, 468-476; Duan et al., Cell., 1997, 89, 555-564; Silva et al., J. Mol. Biol., 2003, 286, 1123-1136; Chevalier et al., Nat. Struct. Biol., 2001, 8, 312-316; Jurica et al., Mol. Cell., 1998, 2, 469-476; Chevalier et al., J. Mol. Biol., 2003, 329, 253-269; Moure et al., J. Mol. Biol., 2003, 334, 685-695; Moure et al., Nat. Struct. Biol., 2002, 9, 764-770; Ichiyanagi et al., J. Mol. Biol., 2000, 300, 889-901; Gimble et al., J. Biol. Chem., 1998, 273, 30524-30529; Bolduc et al., Genes Dev. 2003, 17, 2875-2888; Silva et al., J. Mol. Biol., 1999, 286, 1123-1136; Nakayama et al., J. Mol. Biol., Epub 29 septembre 2006, Spiegel et al., Structure, 2006, 14, 869-880). In contrast with its DNA bound crystal structure (Jurica et al., Mol. Cell., 1998, 2, 469-476; Chevalier et al., Nat. Struct. Biol., 2001, 8, 312-316 ; Chevalier et al., J. Mol. Biol., 2003, 329, 253-269), the structure of I-*Cre*I without bound DNA (Heath et al., Nature Struct. Biol., 1997, 4, 468-476), showed only one I-*Cre*I monomer in the asymmetric unit.

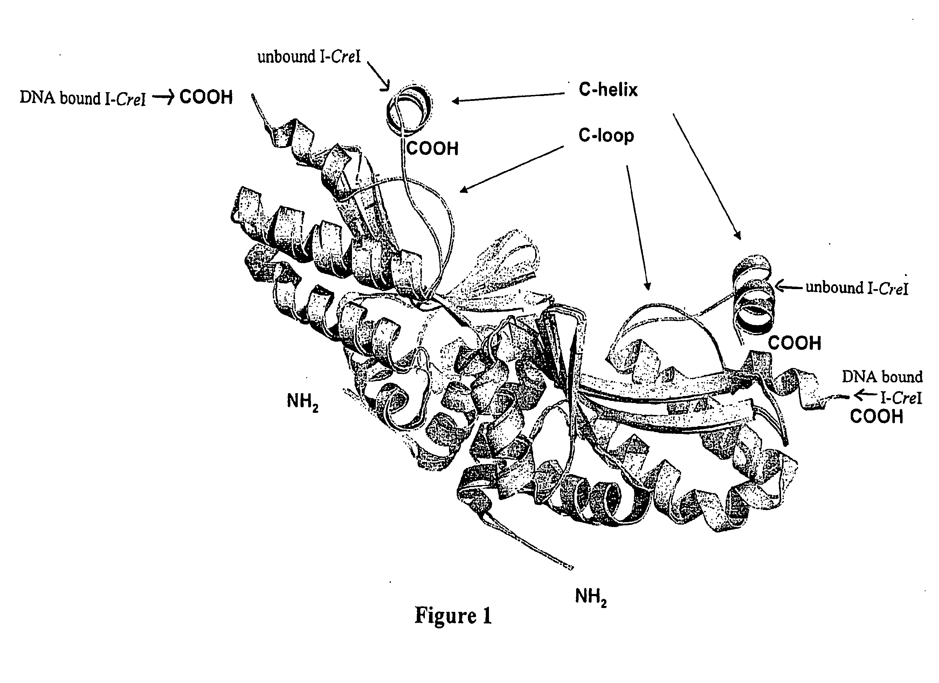

Structural comparisons indicate that LAGLIDADG proteins adopt a similar active conformation and their self-association forms two packed α-helices which separate two monomers or apparent domains. In this core structure (Figure 1), two characteristic αββαββα folds, contributed by two monomers, or two domains in double LAGLIDAG proteins, are facing each other with a two-fold symmetry. On either side of the LAGLIDADG α-helices, a four stranded β-sheet, forming a saddle on the DNA helix major groove, provides a DNA binding interface that drives the interaction of the protein with a half site of the target DNA sequence (Chevalier et al., Nat. Struct. Biol., 2001, 8, 312-316; Jurica et al., Mol. Cell., 1998, 2, 469-476). The catalytic site is central, formed with contributions from helices of both monomers. Just above the catalytic site, the two LAGLIDADG α-helices play also an essential role in the dimerization interface. In addition to this core structure, other domains can be found, for instance, PI-*Sce*I, an intein, has a protein splicing domain, and an additional DNA-binding domain (Moure et al., Nat. Struct. Biol., 2002, 9, 764-770; Pingoud et al., Biochemistry, 1998, 37, 8233-8243).

Modifying the substrate specificity of DNA binding proteins by mutagenesis and screening/selection is a difficult task (Lanio et al., Protein Eng., 2000, 13, 275-281; Voziyanov et al., J. Mol. Biol., 2003, 326, 65-76; Santoro et al., P.N.A.S., 2002, 99, 4185-4190; Buchholz, F. and Stewart, A.F., Nat. Biotechnol., 2001, 19, 1047-1052). This is even harder in the case of HEs whose main characteristic is their large DNA recognition sites.

Analysis of the I-*Cre*I/DNA crystal structure indicates that in each monomer, nine residues (S32, Y33, Q38, N30, K28, Q26, Q44, R68 and R70) establish direct interaction with eight bases at positions ± 3, 4, 5, 6, 7, 9, 10 and 11 of the homing site (Jurica et al., Mol. Cell., 1998, 2, 469-76; Chevalier et al., J. Mol. Biol., 2003, 329, 253-269), which randomization would result in 20⁹ combinations, a number beyond any screening capacity today. In addition, a total of 28 (Left-monomer) or 24 (Right-monomer) water molecules mediate additional contacts between nucleotides and protein side-chains in the protein/DNA interface (Chevalier et al., J. Mol. Biol., 2003, 329, 253-269).

Therefore, several laboratories have relied on a semi-rational approach (Chica et al., Curr. Opin. Biotechnol., 2005, 16, 378-384) to limit the diversity of the mutant libraries to be handled, choosing a small set of relevant amino acid residues according to structural data. This set is generally composed of amino acid residues of the four stranded β-sheet that, in the HE/DNA complex structure, make direct or water-mediated contacts with the nucleotide bases of the homing site.

This semi-rational approach was used to locally alter the specificity of the I-*Cre*I (Seligman et al., Genetics, 1997, 147, 1653-64; Seligman et al., Nucleic Acids Res., 2002, 30, 3870-3879; Sussman et al., J. Mol. Biol., 2004, 342, 31-41; Rosen et al., Nucleic Acids Res., 2006, 34, 4791-4800; Arnould et al., J. Mol. Biol., 2006, 355, 443-458; International PCT Applications WO 2006/097853 and WO 2006/097784; Smith et al., Nucleic Acids Res., Epub 27 November 2006), I-SceI (Doyon et al., J. Am. Chem. Soc., 2006, 128, 2477-2484), PI-*Sce*I (Gimble et al., J. Mol. Biol, 2003, 334, 993-1008) and I*-Mso*I (Ashworth et al., Nature, 2006, 441, 656-659) proteins.

By combining the semi-rational approach and high throughput screening (HTS; Arnould et al., J. Mol. Biol., 2006, 355, 443-458; International PCT Applications WO 2006/097853 and WO 2006/097784; Smith et al., Nucleic Acids Res., Epub 27 November 2006), it was possible to obtain large number of locally altered variants of the I-*Cre*I meganuclease that recognize a variety of targets, and to assemble them by a combinatorial process, to obtain entirely redesigned mutants with chosen specificity.

However, this approach is not easy since the HEs DNA binding interface is very compact and the two different ββ hairpins which are responsible for virtually all base-specific interactions are part of a single fold. Thus, the mutation of several amino acids placed in close vicinity which is required for binding a target mutated at several positions may disrupt the structure of the binding interface.

Therefore, to reach a larger number of sequences, it would be extremely valuable to be able to identify other regions in the LAGLIDAG endonucleases, which can be engineered to generate novel substrate specificity.

In addition, since homing endonucleases can sometimes be harmful at very high doses (Gouble et al., J. Gene Med., 2006, 8, 616-622), it would be extremely valuable to engineer LAGLIDADG endonucleases which are less toxic.

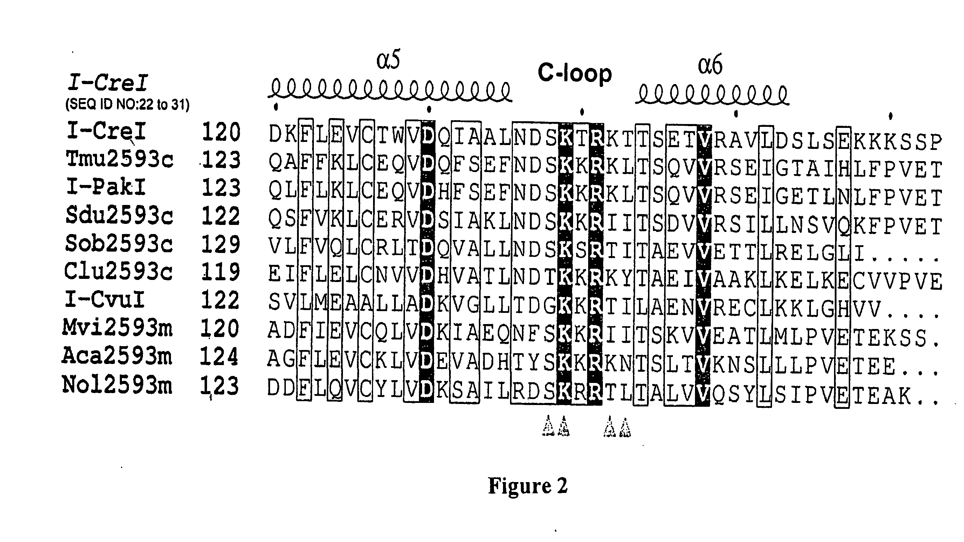

The inventors have solved the structure of the I-*Cre*I dimer without DNA; its comparison with the DNA bound crystal structure (PDB code 1gz9; Chevalier et al., Nat. Struct. Biol., 2001, 8, 312-316) depicts a different conformation of the C-terminal loop and the final helix α6, which suggests its implication in DNA binding. A site-directed mutagenesis study in this region demonstrates that whereas the C-terminal helix is negligible for DNA binding, the final C-terminal loop which is well conserved among homodimeric proteins from the LAGLIDADG family (Figure 2) and makes a number of nonspecific contacts to the DNA phosphate backbone (Jurica et al., Mol. Cell., 1998, 2, 469-76; Chevalier et al., J. Mol. Biol., 2003, 329, 253-269), plays an important role not only in binding and cleavage but also in target specificity. In addition, some of the mutants in the C-terminal loop were significantly less toxic than wild-type I-*Cre*I.

This region open new possibilities to engineer new homing endonucleases having novel substrate specificities and thereby increases the number of DNA sequences that can be targeted with meganucleases. Thus, redesigned meganucleases cleaving chosen genomic targets from genes of interest can be engineered by combining previously identified mutations as defined above (Arnould et al., J. Mol. Biol., 2006, 355, 443-458; International PCT Applications WO 2006/097853, WO 2006/097854 and WO 2006/097784; Smith et al., Nucleic Acids Res., Epub 27 November 2006), with mutations in the final C-terminal loop.

In addition, this region allows also the engineering of homing endonucleases which are less toxic.

Potential applications include genetic engineering, genome engineering, gene therapy and antiviral therapy.

The invention relates to a method for engineering an I-*Cre*I variant having novel substrate specificity, comprising at least the following steps:
(a) the mutation of at least one amino acid residue selected from the group consisting of residues in positions 138 and 142 by reference to I*-Cre*I amino acid sequence numbering of the final C-terminal loop of I-*Cre*I, and
(b) the selection and/or screening of the variants from step (a) having a pattern of cleaved DNA targets that is different from that of the parent I-*Cre*I.

### Definitions

- Amino acid residues in a polypeptide sequence are designated herein according to the one-letter code, in which, for example, Q means Gln or Glutamine residue, R means Arg or Arginine residue and D means Asp or Aspartic acid residue.
- hydrophobic amino acid refers to leucine (L), valine (V), isoleucine (I), alanine (A), methionine (M), phenylalanine (F), tryptophane (W) and tyrosine (Y).
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "meganuclease" is intended an endonuclease having a double-stranded DNA target sequence of 12 to 45 pb.
- by "parent LAGLIDADG homing endonuclease" is intended a wild-type LAGLIDADG homing endonuclease or a functional variant thereof. Said parent LAGLIDADG homing endonuclease may be a monomer, a dimer (homodimer or heterodimer) comprising two LAGLIDADG homing endonuclease core domains which are associated in a functional endonuclease able to cleave a double-stranded DNA target of 22 to 24 bp.
- by "homodimeric LAGLIDADG "homing endonuclease" is intended a wild-type homodimeric LAGLIDADG homing endonuclease having a single LAGLIDADG motif and cleaving palindromic DNA target sequences, such as I-*Cre*I or I*-Mso*I or a functional variant thereof.
- by "LAGLIDADG homing endonuclease variant" or "variant" is intended a protein obtained by replacing at least one amino acid of a LAGLIDADG homing endonuclease sequence, with a different amino acid.
- by "functional variant" is intended a LAGLIDADG homing endonuclease variant which is able to cleave a DNA target, preferably a new DNA target which is not cleaved by a wild-type LAGLIDADG homing endonuclease. For example, such variants have amino acid variation at positions contacting the DNA target sequence or interacting directly or indirectly with said DNA target.
- by "homing endonuclease variant with novel specificity" is intended a variant having a pattern of cleaved targets (cleavage profile) different from that of the parent homing endonuclease. The variants may cleave less targets (restricted profile) or more targets than the parent homing endonuclease. Preferably, the variant is able to cleave at least one target that is not cleaved by the parent homing endonuclease.

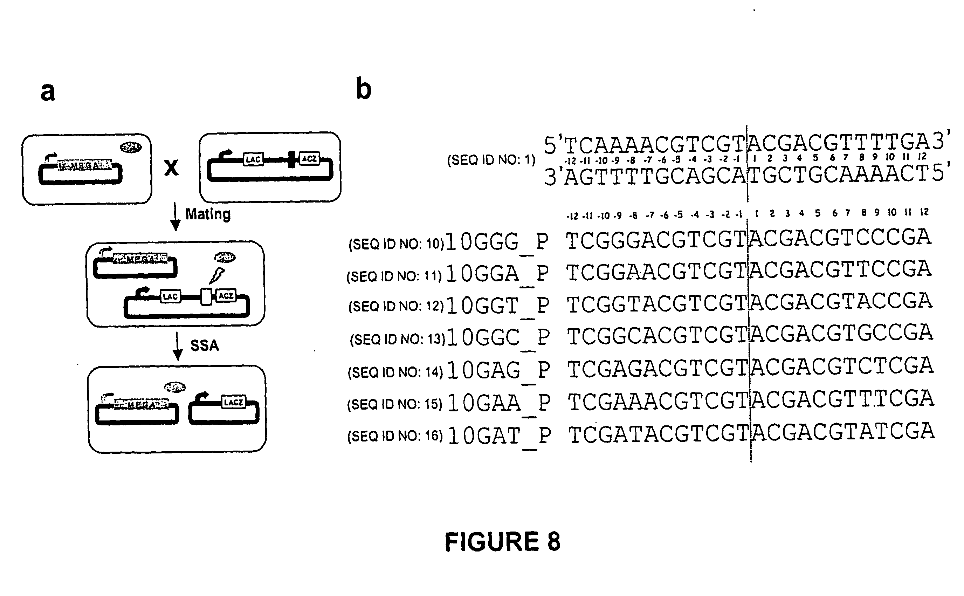

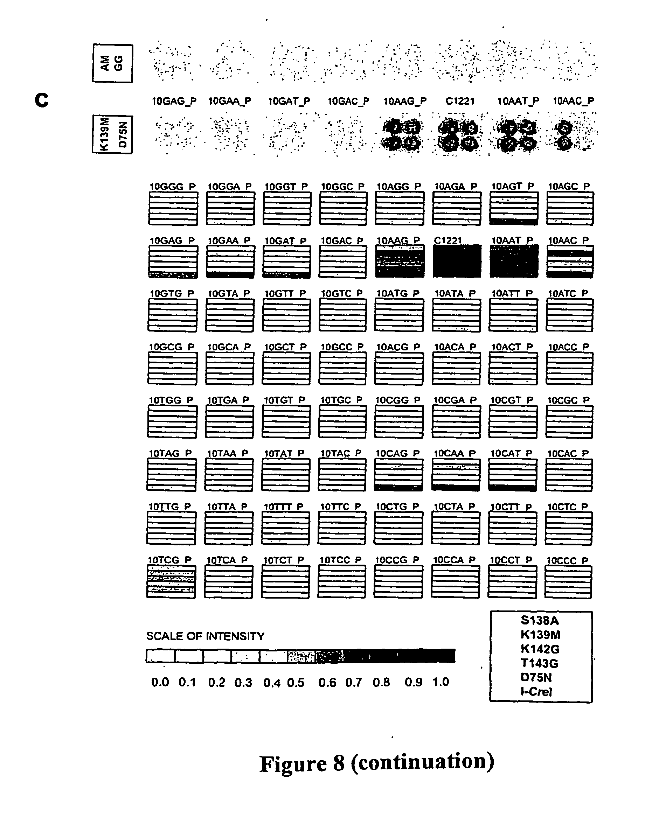

The terms "novel specificity", "modified specificity", "novel cleavage specificity", "novel substrate specificity" which are equivalent and used indifferently, refer to the specificity of the variant towards the nucleotides of the DNA target sequence.
- by "I*-Cre*I" is intended the wild-type I-*Cre*I having the sequence SWISSPROT P05725 or pdb accession code 1g9y.
- by "domain" or "core domain" is intended the "LAGLIDADG homing endonuclease core domain" which is the characteristic α₁β₁β₂α₂β₃β₄α₃ fold of the homing endonucleases of the LAGLIDADG family, corresponding to a sequence of about one hundred amino acid residues. Said domain comprises four beta-strands (β₁, β₂, β₃, β₄) folded in an antiparallel beta-sheet which interacts with one half of the DNA target. This domain is able to associate with another LAGLIDADG homing endonuclease core domain which interacts with the other half of the DNA target to form a functional endonuclease able to cleave said DNA target. For example, in the case of the dimeric homing endonuclease I-*Cre*I (163 amino acids), the LAGLIDADG homing endonuclease core domain corresponds to the residues 6 to 94. In the case of monomeric homing endonucleases, two such domains are found in the sequence of the endonuclease; for example in I*-Dmo*I (194 amino acids), the first domain (residues 7 to 99) and the second domain (residues 104 to 194) are separated by a short linker (residues 100 to 103).
- by "subdomain" is intended the region of a LAGLIDADG homing endonuclease core domain which interacts with a distinct part of a homing endonuclease DNA target half-site. Two different subdomains behave independently and the mutation in one subdomain does not alter the binding and cleavage properties of the other subdomain. Therefore, two subdomains bind distinct part of a homing endonuclease DNA target half-site.
- by "beta-hairpin" is intended two consecutive beta-strands of the antiparallel beta-sheet of a LAGLIDADG homing endonuclease core domain (β₁β₂ or, β₃β₄) which are connected by a loop or a turn,
- by "DNA target", "DNA target sequence", "target sequence" , "target-site", "target" , "site"; "recognition site", "recognition sequence", "homing recognition site", "homing site", "cleavage site" is intended a 22 to 24 bp double-stranded palindromic, partially palindromic (pseudo-palindromic) or non-palindromic polynucleotide sequence that is recognized and cleaved by a LAGLIDADG homing endonuclease. These terms refer to a distinct DNA location, preferably a genomic location, at which a double stranded break (cleavage) is to be induced by the endonuclease. The DNA target is defined by the 5' to 3' sequence of one strand of the double-stranded polynucleotide. For example, the palindromic DNA target sequence cleaved by wild-type I*-Cre*I presented in figure 8 is defined by the sequence 5'- t₋₁₂c₋₁₁a₋₁₀a₋₉a₋₈a₋₇c₋₆g₋₅t₋₄c₋₃g₋₂t₋₁a₊₁c₊₂g₊₃a₊₄c₊₅g₊₆t₊₇t₊₈t₊₉t₊₁₀g₊₁₁a₊₁₂ (SEQ ID NO:1). Cleavage of the DNA target occurs at the nucleotides in positions +2 and -2, respectively for the sense and the antisense strand. Unless otherwise indicated, the position at which cleavage of the DNA target by an I*-Cre* I meganuclease variant occurs, corresponds to the cleavage site on the sense strand of the DNA target.
- by " DNA target half-site", "half cleavage site" or half-site" is intended the portion of the DNA target which is bound by each LAGLIDADG homing endonuclease core domain.
- by "chimeric DNA target" or "hybrid DNA target" is intended the fusion of a different half of two parent meganuclease target sequences. In addition at least one half of said target may comprise the combination of nucleotides which are bound by separate subdomains (combined DNA target).
- by "vector" is intended a nucleic acid molecule capable of transporting another nucleic acid to which it has been linked.
- by "mutation" is intended the substitution, the deletion, and/or the addition of one or more nucleotides/amino acids in a nucleic acid/amino acid sequence.
- by "homologous" is intended a sequence with enough identity to another one to lead to a homologous recombination between sequences, more particularly having at least 95 % identity, preferably 97 % identity and more preferably 99 %.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and ruminants (e.g., cows, pigs, horses).
- "genetic disease" refers to any disease, partially or completely, directly or indirectly, due to an abnormality in one or several genes. Said abnormality can be a mutation, an insertion or a deletion. Said mutation can be a punctual mutation. Said abnormality can affect the coding sequence of the gene or its regulatory sequence. Said abnormality can affect the structure of the genomic sequence or the structure or stability of the encoded mRNA. Said genetic disease can be recessive or dominant. Such genetic disease could be, but are not limited to, cystic fibrosis, Huntington's chorea, familial hyperchoiesterolemia (LDL receptor defect), hepatoblastoma, Wilson's disease, congenital hepatic porphyrias, inherited disorders of hepatic metabolism, Lesch Nyhan syndrome, sickle cell anemia, thalassaemias, xeroderma pigmentosum, Fanconi's anemia, retinitis pigmentosa, ataxia telangiectasia, Bloom's syndrome, retinoblastoma, Duchenne's muscular dystrophy, and Tay-Sachs disease.

According to the invention, the amino acids of the final C-terminal loop correspond to positions 137 to 143 in I-*Cre*I amino acid sequence SEQ ID NO: 2 or Swissprot P05725. Knowing the positions of the final C-terminal loop in I*-Cre*I, one skilled in the art can easily deduce the corresponding positions in another homodimeric LAGLIDADG homing endonuclease, using well-known protein structure analyses softwares such as Pymol. For example, for I*-Mso*I, the final C-terminal loop corresponds to positions 143 to 149. According to an advantageous embodiment of said method, step (a) comprises the mutation of amino acid residue(s) of the final C-terminal loop that are contacting the phosphate backbone of the parent LAGLIDADG endonuclease DNA cleavage site (wild-type LAGLIDAG endonuclease homing site). Preferably said residues are involved in binding and cleavage of said DNA cleavage site. More preferably, said residues are in positions 138, 139, 142 or 143, by reference to the numbering of *I-CreI* amino acid sequence (SEQ ID NO: 2; figure 2). Two residues may be mutated in one variant provided that each mutation is in a different pair of residues chosen from the pair of residues in positions 138 and 139 and the pair of residues in positions 142 and 143.

According to the method of the invention, the mutations which are introduced modify the interaction(s) of said amino acid(s) of the final C-terminal loop with the phosphate backbone of the parent LAGLIDADG endonuclease DNA cleavage site.

According to another advantageous embodiment of said method, the mutation in step (a) is a substitution of at least one amino acid of said final C-terminal loop, with a different amino acid.

Preferably, the residue in position 138 or 139 is substituted by an hydrophobic amino acid to avoid the formation of hydrogen bonds with the phosphate backbone of the DNA cleavage site. For example, the residue in position 138 is substituted by an alanine or the residue in position 139 is substituted by a methionine.

The residue in position 142 or 143 is advantageously substituted by a small amino acid, for example a glycine, to decrease the size of the side chains of these amino acid residues.

According to the method of the invention, the mutation(s) in step (a) are introduced in either a wild-type LAGLIDADG homing endonuclease or a functional variant thereof.

The wild-type LAGLIDADG homing endonuclease is advantageously homodimeric. Examples of wild-type homodimeric LAGLIDAG homing endonucleases are presented in Table 1 of Lucas et al., Nucleic Acids Res., 2001, 29, 960-969. The wild-type homodimeric LAGLIDADG homing endonuclease may be advantageously selected from the group consisting of: I-*Cre*I, I*-Ceu*I, I-*Mso*I and I-*Cpa*I, preferably I-*Cre*I.

The functional variant comprises additional mutations outside the final C-terminal loop, preferably in positions of amino acid residues which interact with a DNA target half-site. The LAGLIDADG homing endonucleases DNA interacting residues are well-known in the art. The residues which are mutated may interact with the DNA backbone or with the nucleotide bases, directly or via a water molecule. Preferably said mutations modify the cleavage specificity of the meganuclease and result in a meganuclease with novel specificity, which is able to cleave a DNA target from a gene of interest. More preferably, said mutations are substitutions of one or more amino acids in a first functional subdomain corresponding to that situated from positions 26 to 40 of I-*Cre*I amino acid sequence, that alter the specificity towards the nucleotide in positions ± 8 to 10 of the DNA target, and/or substitutions in a second functional subdomain corresponding to that situated from positions 44 to 77 of I-*Cre*I amino acid sequence, that alter the specificity towards the nucleotide in positions ± 3 to 5 of the DNA target, as described previously (International PCT Applications WO 2006/097784 and WO 2006/097853; Arnould et al., J. Mol. Biol., 2006, 355, 443-458; Smith et al., Nucleic Acids Res., 2006). The substitutions correspond advantageously to positions 26, 28, 30, 32, 33, 38, and/or 40, 44, 68, 70, 75 and/or 77 of I-*Cre*I amino acid sequence. For cleaving a DNA target, wherein n₋₄ is t or n₊₄ is a, said variant has advantageously a glutamine (Q) in position 44; for cleaving a DNA target, wherein n₋₄ is a or n₊₄ is t, said variant has an alanine (A) or an asparagine in position 44, and for cleaving a DNA target, wherein n₋₉ is g or n₊₉ is c, said variant has advantageously an arginine (R) or a lysine (K) in position 38.

According to a most preferred embodiment of said method, the parent LAGLIDADG homing endonuclease is an I-*Cre*I variant having mutations in positions 26 to 40 and 44 to 77 of I*-Cre*I and cleaving a palindromic DNA sequence, wherein at least the nucleotides in positions +3 to +5 and +8 to +10 or -10 to -8 and -5 to -3 of one half of said DNA sequence correspond to the nucleotides in positions +3 to +5 and +8 to +10 or -10 to -8 and -5 to -3 of one half of a DNA target from a gene of interest.

The mutations in step (a) are introduced according to standard mutagenesis methods which are well-known in the art and commercially available. They may be advantageously produced by amplifying overlapping fragments comprising the mutated position(s), as defined above, according to well-known overlapping PCR techniques. Libraries of variants having amino acid variation in the final C-terminal loop may be generated according to standard methods.

Step (a) may comprise the introduction of additional mutations at other positions contacting the DNA target sequence or interacting directly or indirectly with said DNA target, as defined above. This step may be performed by generating combinatorial libraries as described in the International PCT Application WO 2004/067736, Arnould et al., J. Mol. Biol., 2006, 355, 443-458 and Smith et al., Nucleic Acids Res., Epub 27 November 2006 and eventually, combining said mutations intramolecularly, by amplifying overlapping fragments comprising each of the mutations, according to well-known overlapping PCR techniques.

Furthermore, random mutations may also be introduced on the whole variant or in part of the variant, in particular the C-terminal half of the variant (positions 80 to 163 of I-*Cre*I amino acid sequence SEQ ID NO:2) in order to improve the binding and/or cleavage properties of the variant towards a DNA target from a gene of interest.

The additional mutations (random or site-specific) and the mutation(s) in the final C-terminal loop may be introduced simultaneously or subsequently.

In addition, one or more residues may be inserted at the NH₂ terminus and/or COOH terminus of the variant monomer(s)/domain(s). For example, a methionine residue is introduced at the NH₂ terminus, a tag (epitope or polyhistidine sequence) is introduced at the NH₂ terminus and/or COOH terminus; said tag is useful for the detection and/or the purification of the meganuclease.

The selection and/or screening in step (b) may be performed by using a cleavage assay *in vitro* or *in vivo,* as described in the International PCT Application WO 2004/067736.

According to another advantageous embodiment of said method, step (b) is performed *in vivo,* under conditions where the double-strand break in a mutated DNA target sequence which is generated by said variant leads to the activation of a positive selection marker or a reporter gene, or the inactivation of a negative selection marker or a reporter gene, by recombination-mediated repair of said DNA double-strand break.

For example, the cleavage activity of the variant of the invention may be measured by a direct repeat recombination assay, in yeast or mammalian cells, using a reporter vector, as described in the PCT Application WO 2004/067736; Epinat et al., Nucleic Acids Res., 2003, 31, 2952-2962; Chames et al., Nucleic Acids Res., 2005, 33, e178, and Arnould et al., J. Mol. Biol., 2006, 355, 443-458. The reporter vector comprises two truncated, non-functional copies of a reporter gene (direct repeats) and a chimeric DNA target sequence within the intervening sequence, cloned in a yeast or a mammalian expression vector. The DNA target sequence is derived from the parent homing endonuclease cleavage site by replacement of at least one nucleotide by a different nucleotide. Preferably a panel of palindromic or non-palindromic DNA targets representing the different combinations of the 4 bases (g, a, c, t) at one or more positions of the DNA cleavage site is tested (4" palindromic targets for n mutated positions). Expression of the variant results in a functional endonuclease which is able to cleave the DNA target sequence. This cleavage induces homologous recombination between the direct repeats, resulting in a functional reporter gene, whose expression can be monitored by appropriate assay.

According to another advantageous embodiment of said method, step (b) comprises the selection and/or screening of the variants from step (a) which are able to cleave at least one DNA target sequence that is not cleaved by said parent LAGLIDADG homing endonuclease, said DNA target sequence being derived from the parent LAGLIDADG homing endonuclease cleavage site, by the replacement of at least one nucleotide of one half of said cleavage site, with a different nucleotide.

According to the method of the invention, the parent DNA target may be palindromic, non-palindromic or pseudo-palindromic. Preferably, said DNA target sequence is derived from the I-*C*reI palindromic site having the sequence SEQ ID NO: 1. More preferably, said DNA target has nucleotide mutation(s) in positions ± 1 to 2, ± 6 to 7, ± 8 to 10 and/or ± 11 to 12, still more preferably in positions ± 1 to 2, ± 6 to 7 and/or ± 11 to 12.

According to another advantageous embodiment of said method, it comprises a further step (c) of expressing one variant obtained in step (b), so as to allow the formation of homodimers. Said homodimers are able to cleave a palindromic or pseudo-palindromic target sequences.

According to another advantageous embodiment of said method, it comprises a further step (c') of co-expressing one variant obtained in step (b) and a wild-type LAGLIDADG homing endonuclease or a functional variant thereof, so as to allow the formation of heterodimers. The assembly of functional heterodimers by co-expression of two different LAGLIDADG endonucleases monomers, has been described previously in Arnould et al., J. Mol. Biol., 2006, 355, 443-458; International PCT Applications WO 2006/097853, WO 2006/097854 and WO 2006/097784; Smith et al., Nucleic Acids Res., Epub 27 November 2006. Preferably, two different variants obtained in step (b) are co-expressed. Said heterodimers are able to cleave a non-palindromic chimeric target.

For example, host cells may be modified by one or two recombinant expression vector(s) encoding said variant(s). The cells are then cultured under conditions allowing the expression of the variant(s) and the homodimers/heterodimers which are formed are then recovered from the cell culture.

According to the method of the invention, single-chain chimeric meganucleases may be constructed by the fusion of one variant obtained in step (b) with a homing endonuclease domain/monomer. Said domain/monomer may be from a wild-type LAGLIDADG homing endonuclease or a functional variant thereof. Preferably, the two domain(s)/monomer(s) are connected by a peptidic linker. More preferably, the single-chain meganuclease comprises two different variants obtained in step (b); said single-chain meganuclease is able cleave a non-palindromic chimeric target comprising one different half of each variant DNA target.

Methods for constructing single-chain chimeric meganucleases derived from homing endonucleases are well-known in the art (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). Any of such methods, may be applied for constructing single-chain chimeric meganucleases derived from the variants as defined in the present invention.

The invention relates also to an homodimeric or heterodimeric LAGLIDADG homing endonuclease variant obtainable by the method as defined above, with the exclusion of the homodimeric variants of SEQ ID NO: 3 and 4 and the homodimeric or heterodimeric variants comprising a monomer of SEQ ID NO: 5; the LAGLIDADG homing endonuclease variant of the invention is also named as variant, meganuclease variant or meganuclease.

According to an advantageous embodiment of said variant, it is an heterodimer comprising monomers from two different variants obtainable by the method as defined above.

According to another advantageous embodiment of said variant, it is an I-*Cre*I variant having one or two mutations, each one from a different pair of mutations selected from the group consisting of the pair S138A and K139M and the pair K142G and T143G. Examples of such variants include SEQ ID NO: 6 to 9.

More preferably, said I-*Cre*I variant is an heterodimer, comprising two monomers, each one further comprising different mutations in positions 26 to 40 and 44 to 77 of I*-Cre*I and being able to cleave a genomic DNA target from a gene of interest.

The subject-matter of the present invention is also a single-chain chimeric meganuclease derived from the variant as defined above; the single-chain chimeric meganuclease of the invention is also named as single-chain derivative, single-chain meganuclease, single-chain meganuclease derivative or meganuclease.

The meganuclease of the invention includes both the meganuclease variant and the single-chain meganuclease derivative.

The subject-matter of the present invention is also a polynucleotide fragment encoding a variant or a single-chain derivative as defined above; said polynucleotide may encode one monomer of an homodimeric or heterodimeric variant, or two domains/monomers of a single-chain derivative.

The subject-matter of the present invention is also a recombinant vector for the expression of a variant or a single-chain derivative according to the invention. The recombinant vector comprises at least one polynucleotide fragment encoding a variant or a single-chain meganuclease, as defined above. In a preferred embodiment, said vector comprises two different polynucleotide fragments, each encoding one of the monomers of an heterodimeric variant.

A vector which can be used in the present invention includes, but is not limited to, a viral vector, a plasmid, a RNA vector or a linear or circular DNA or RNA molecule which may consists of a chromosomal, non chromosomal, semisynthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), para-myxovirus (e. g. measles and Sendai), positive strand RNA viruses such as picor-navirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosissarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

Preferred vectors include lentiviral vectors, and particularly self inactivacting lentiviral vectors.

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; TRP1 for *S*. *cerevisiae;* tetracycline, rifampicin or ampicillin resistance in *E*. *coli.*

Preferably said vectors are expression vectors, wherein the sequence(s) encoding the variant/single-chain derivative of the invention is placed under control of appropriate transcriptional and translational control elements to permit production or synthesis of said meganuclease. Therefore, said polynucleotide is comprised in an expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said variant is an heterodimer, the two polynucleotides encoding each of the monomers are included in one vector which is able to drive the expression of both polynucleotides, simultaneously. Suitable promoters include tissue specific and/or inducible promoters. Examples of inducible promoters are: eukaryotic metallothionine promoter which is induced by increased levels of heavy metals, prokaryotic lacZ promoter which is induced in response to isopropyl-β-D-thiogalacto-pyranoside (IPTG) and eukaryotic heat shock promoter which is induced by increased temperature. Examples of tissue specific promoters are skeletal muscle creatine kinase, prostate-specific antigen (PSA), α-antitrypsin protease, human surfactant (SP) A and B proteins, β-casein and acidic whey protein genes.

According to another advantageous embodiment of said vector, it includes a targeting DNA construct comprising sequences sharing homologies with the region surrounding the genomic DNA target cleavage site as defined above.

Alternatively, the vector coding for the meganuclease and the vector comprising the targeting DNA construct are different vectors.

More preferably, the targeting DNA construct comprises:
a) sequences sharing homologies with the region surrounding the genomic DNA cleavage site as defined above, and
b) a sequence to be introduced flanked by sequences as in a).

Preferably, homologous sequences of at least 50 bp, preferably more than 100 bp and more preferably more than 200 bp are used. Indeed, shared DNA homologies are located in regions flanking upstream and downstream the site of the break and the DNA sequence to be introduced should be located between the two arms. The sequence to be introduced is preferably a sequence which repairs a mutation in the gene of interest (gene correction or recovery of a functional gene), for the purpose of genome therapy. Alternatively, it can be any other sequence used to alter the chromosomal DNA in some specific way including a sequence used to modify a specific sequence, to attenuate or activate the endogenous gene of interest, to inactivate or delete the endogenous gene of interest or part thereof, to introduce a mutation into a site of interest or to introduce an exogenous gene or part thereof.

The invention also concerns a prokaryotic or eukaryotic host cell which is modified by a polynucleotide or a vector as defined above, preferably an expression vector.

The invention also concerns a non-human transgenic animal or a transgenic plant, characterized in that all or part of their cells are modified by a polynucleotide or a vector as defined above.

As used herein, a cell refers to a prokaryotic cell, such as a bacterial cell, or eukaryotic cell, such as an animal, plant or yeast cell.

The subject-matter of the present invention is further the use of a meganuclease with the exclusion of SEQ ID NO: 5, one or two derived polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering, for non-therapeutic purposes.

Non therapeutic purposes include for example (i) gene targeting of specific loci in cell packaging lines for protein production, (ii) gene targeting of specific loci in crop plants, for strain improvements and metabolic engineering, (iii) targeted recombination for the removal of markers in genetically modified crop plants, (iv) targeted recombination for the removal of markers in genetically modified microorganism strains (for antibiotic production for example).

According to an advantageous embodiment of said use, it is for inducing a double-strand break in a site of interest comprising a DNA target sequence, thereby inducing a DNA recombination event, a DNA loss or cell death.

According to the invention, said double-strand break is for: repairing a specific sequence, modifying a specific sequence, restoring a functional gene in place of a mutated one, attenuating or activating an endogenous gene of interest, introducing a mutation into a site of interest, introducing an exogenous gene or a part thereof, inactivating or detecting an endogenous gene or a part thereof, translocating a chromosomal arm, or leaving the DNA unrepaired and degraded.

The subject-matter of the present invention is also a method of genetic engineering, characterized in that it comprises a step of double-strand nucleic acid breaking in a site of interest located on a vector comprising a DNA target as defined hereabove, by contacting said vector with a meganuclease as defined above, with the exclusion of SEQ ID NO: 5, thereby inducing an homologous recombination with another vector presenting homology with the sequence surrounding the cleavage site of said meganuclease.

The subjet-matter of the present invention is also a method of genome engineering, characterized in that it comprises the following steps: 1) double-strand breaking a genomic locus comprising at least one DNA target of a meganuclease as defined above, by contacting said target with said meganuclease, with the exclusion of SEQ ID NO: 5 ; 2) maintaining said broken genomic locus under conditions appropriate for homologous recombination with a targeting DNA construct comprising the sequence to be introduced in said locus, flanked by sequences sharing homologies with the targeted locus.

The subject-matter of the present invention is also a method of genome engineering, characterized in that it comprises the following steps: 1) double-strand breaking a genomic locus comprising at least one DNA target of a meganuclease as defined above, by contacting said cleavage site with said meganuclease, with the exclusion of SEQ ID NO: 5; 2) maintaining said broken genomic locus under conditions appropriate for homologous recombination with chromosomal DNA sharing homologies to regions surrounding the cleavage site.

The subject-matter of the present invention is also the use of at least one meganuclease as defined above, with the exclusion of SEQ ID NO: 5, one or two derived polynucleotide(s), preferably included in expression vector(s), as defined above, for the preparation of a medicament for preventing, improving or curing a genetic disease in an individual in need thereof, said medicament being administrated by any means to said individual.

The subject-matter of the present invention is also a method for preventing, improving or curing a genetic disease in an individual in need thereof, said method comprising the step of administering to said individual a composition comprising at least a meganuclease as defined above, by any means.

In this case, the use of the meganuclease as defined above, comprises at least the step of (a) inducing in somatic tissue(s) of the individual a double stranded cleavage at a site of interest of a gene comprising at least one recognition and cleavage site of said meganuclease, and (b) introducing into the individual a targeting DNA, wherein said targeting DNA comprises (1) DNA sharing homologies to the region surrounding the cleavage site and (2) DNA which repairs the site of interest upon recombination between the targeting DNA and the chromosomal DNA. The targeting DNA is introduced into the individual under conditions appropriate for introduction of the targeting DNA into the site of interest.

According to the present invention, said double-stranded cleavage is induced, either *in toto* by administration of said meganuclease to an individual, or *ex vivo* by introduction of said meganuclease into somatic cells removed from an individual and returned into the individual after modification.

In a preferred embodiment of said use, the meganuclease is combined with a targeting DNA construct comprising a sequence which repairs a mutation in the gene flanked by sequences sharing homologies with the regions of the gene surrounding the genomic DNA cleavage site of said meganuclease, as defined above. The sequence which repairs the mutation is either a fragment of the gene with the correct sequence or an exon knock-in construct.

For correcting a gene, cleavage of the gene occurs in the vicinity of the mutation, preferably, within 500 bp of the mutation. The targeting construct comprises a gene fragment which has at least 200 bp of homologous sequence flanking the genomic DNA cleavage site (minimal repair matrix) for repairing the cleavage, and includes the correct sequence of the gene for repairing the mutation. Consequently, the targeting construct for gene correction comprises or consists of the minimal repair matrix; it is preferably from 200 pb to 6000 pb, more preferably from 1000 pb to 2000 pb.

For restoring a functional gene, cleavage of the gene occurs upstream of a mutation. Preferably said mutation is the first known mutation in the sequence of the gene, so that all the downstream mutations of the gene can be corrected simultaneously. The targeting construct comprises the exons downstream of the genomic DNA cleavage site fused in frame (as in the cDNA) and with a polyadenylation site to stop transcription in 3'. The sequence to be introduced (exon knock-in construct) is flanked by introns or exons sequences surrounding the cleavage site, so as to allow the transcription of the engineered gene (exon knock-in gene) into a mRNA able to code for a functional protein. For example, the exon knock-in construct is flanked by sequences upstream and downstream.

The subject-matter of the present invention is also the use of at least one meganuclease as defined above, with the exclusion of SEQ ID NO: 5, one or or two derived polynucleotide(s), preferably included in expression vector(s), as defined above for the preparation of a medicament for preventing, improving or curing a disease caused by an infectious agent that presents a DNA intermediate, in an individual in need thereof, said medicament being administrated by any means to said individual.

The subject-matter of the present invention is also a method for preventing, improving or curing a disease caused by an infectious agent that presents a DNA intermediate, in an individual in need thereof, said method comprising at least the step of administering to said individual a composition as defined above, by any means.

The subject-matter of the present invention is also the use of at least one meganuclease as defined above, one or two polynucleotide(s), preferably included in expression vector(s), as defined above, *in vitro,* for inhibiting the propagation, inactivating or deleting an infectious agent that presents a DNA intermediate, in biological derived products or products intended for biological uses or for disinfecting an object.

The subject-matter of the present invention is also a method for decontaminating a product or a material from an infectious agent that presents a DNA intermediate, said method comprising at least the step of contacting a biological derived product, a product intended for biological use or an object, with a composition as defined above, for a time sufficient to inhibit the propagation, inactivate or delete said infectious agent.

In a particular embodiment, said infectious agent is a virus. For example said virus is an adenovirus (Ad11, Ad21), herpesvirus (HSV, VZV, EBV, CMV, herpesvirus 6, 7 or 8), hepadnavirus (HBV), papovavirus (HPV), poxvirus or retrovirus (HTLV, HIV).

The subject-matter of the present invention is also a composition characterized in that it comprises at least one meganuclease with the exclusion of SEQ ID NO:5, one or two derived polynucleotide(s), preferably included in expression vector(s), as defined above.

In a preferred embodiment of said composition, it comprises a targeting DNA construct comprising the sequence which repairs the site of interest flanked by sequences sharing homologies with the targeted locus as defined above. Preferably, said targeting DNA construct is either included in a recombinant vector or it is included in an expression vector comprising the polynucleotide(s) encoding the meganuclease, as defined in the present invention.

The subject-matter of the present invention is also products containing at least a meganuclease with the exclusion of SEQ ID NO: 5, or one or two expression vector(s) encoding said meganuclease, and a vector including a targeting construct, as defined above, as a combined preparation for simultaneous, separate or sequential use in the prevention or the treatment of a genetic disease.

For purposes of therapy, the meganuclease and a pharmaceutically acceptable excipient are administered in a therapeutically effective amount. Such a combination is said to be administered in a "therapeutically effective amount" if the amount administered is physiologically significant. An agent is physiologically significant if its presence results in a detectable change in the physiology of the recipient. In the present context, an agent is physiologically significant if its presence results in a decrease in the severity of one or more symptoms of the targeted disease and in a genome correction of the lesion or abnormality.

In one embodiment of the uses according to the present invention, the meganuclease is substantially non-immunogenic, i.e., engenders little or no adverse immunological response. A variety of methods for ameliorating or eliminating deleterious immunological reactions of this sort can be used in accordance with the invention. In a preferred embodiment, the meganuclease is substantially free of N-formyl methionine. Another way to avoid unwanted immunological reactions is to conjugate meganucleases to polyethylene glycol ("PEG") or polypropylene glycol ("PPG") (preferably of 500 to 20,000 daltons average molecular weight (MW)). Conjugation with PEG or PPG, as described by Davis et al. (US 4,179,337) for example, can provide non-immunogenic, physiologically active, water soluble endonuclease conjugates with anti-viral activity. Similar methods also using a polyethylene-polypropylene glycol copolymer are described in Saifer et al. (US 5,006,333).

The meganuclease can be used either as a polypeptide or as a polynucleotide construct/vector encoding said polypeptide. It is introduced into cells, *in vitro*, *ex vivo* or *in vivo,* by any convenient means well-known to those in the art, which are appropriate for the particular cell type, alone or in association with either at least an appropriate vehicle or carrier and/or with the targeting DNA. Once in a cell, the meganuclease and if present, the vector comprising targeting DNA and/or nucleic acid encoding a meganuclease are imported or translocated by the cell from the cytoplasm to the site of action in the nucleus.

The meganuclease (polypeptide) may be advantageously associated with: liposomes, polyethyleneimine (PEI), and/or membrane translocating peptides (Bonetta, The Scientist, 2002, 16, 38; Ford et al., Gene Ther., 2001, 8, 1-4 ; Wadia and Dowdy, Curr. Opin. Biotechnol., 2002, 13, 52-56); in the latter case, the sequence of the meganuclease fused with the sequence of a membrane translocating peptide (fusion protein).

Vectors comprising targeting DNA and/or nucleic acid encoding a meganuclease can be introduced into a cell by a variety of methods (e.g., injection, direct uptake, projectile bombardment, liposomes, electroporation). Meganucleases can be stably or transiently expressed into cells using expression vectors. Techniques of expression in eukaryotic cells are well known to those in the art. (See Current Protocols in Human Genetics: Chapter 12 "Vectors For Gene Therapy" & Chapter 13 "Delivery Systems for Gene Therapy"). Optionally, it may be preferable to incorporate a nuclear localization signal into the recombinant protein to be sure that it is expressed within the nucleus.

The uses of the meganuclease and the methods of using said meganucleases according to the present invention include also the use of the polynucleotide(s), vector(s), cell, transgenic plant or non-human transgenic mammal encoding said meganuclease, as defined above.

According to another advantageous embodiment of the uses and methods according to the present invention, said meganuclease, polynucleotide(s), vector(s), cell, transgenic plant or non-human transgenic mammal are associated with a targeting DNA construct as defined above. Preferably, said vector encoding the monomer(s) of the meganuclease, comprises the targeting DNA construct, as defined above.

The invention concerns also a first method for engineering I-*Cre*I variants able to cleave a genomic DNA target sequence from a gene of interest, comprising at least the steps of:
(a₁) constructing a first series of variants having at least one substitution in a first functional subdomain of the LAGLIDADG core domain situated from positions 26 to 40 of I*-Cre*I,
(b₁) constructing a second series of I-*Cre*I variants having at least one substitution in a second functional subdomain of the LAGLIDADG core domain situated from positions 44 to 77 of I-*Cre*I,
(c₁) selecting and/or screening the variants from the first series of step (a₁) which are able to cleave a mutant I-*Cre*I site wherein (i) the nucleotide triplet in positions -10 to -8 of the I-*Cre*I site has been replaced with the nucleotide triplet which is present in positions -10 to -8 of said genomic target and (ii) the nucleotide triplet in positions +8 to +10 has been replaced with the reverse complementary sequence of the nucleotide triplet which is present in positions -10 to -8 of said genomic target,
(d₁) selecting and/or screening the variants from the second series of step (b₁) which are able to cleave a mutant I-*Cre*I site wherein (i) the nucleotide triplet in positions -5 to -3 of the I*-Cre*I site has been replaced with the nucleotide triplet which is present in positions -5 to -3 of said genomic target and (ii) the nucleotide triplet in positions +3 to +5 has been replaced with the reverse complementary sequence of the nucleotide triplet which is present in positions -5 to -3 of said genomic target,
(e₁) selecting and/or screening the variants from the first series of step (a₁) which are able to cleave a mutant I*-Cre*I site wherein (i) the nucleotide triplet in positions +8 to +10 of the I-*Cre*I site has been replaced with the nucleotide triplet which is present in positions +8 to +10 of said genomic target and (ii) the nucleotide triplet in positions -10 to -8 has been replaced with the reverse complementary sequence of the nucleotide triplet which is present in positions +8 to +10 of said genomic target,
(f₁) selecting and/or screening the variants from the second series of step (b) which are able to cleave a mutant I-*Cre*I site wherein (i) the nucleotide triplet in positions +3 to +5 of the *I-CreI* site has been replaced with the nucleotide triplet which is present in positions +3 to +5 of said genomic target and (ii) the nucleotide triplet in positions -5 to -3 has been replaced with the reverse complementary sequence of the nucleotide triplet which is present in positions +3 to +5 of said genomic target,
(g₁) combining in a single variant, the mutation(s) in positions 26 to 40 and 44 to 77 of two variants from step (c₁) and step (d₁), to obtain a novel homodimeric I*-Cre*I variant which cleaves a sequence wherein (i) the nucleotide triplet in positions -10 to -8 is identical to the nucleotide triplet which is present in positions -10 to -8 of said genomic target, (ii) the nucleotide triplet in positions +8 to +10 is identical to the reverse complementary sequence of the nucleotide triplet which is present in positions -10 to -8 of said genomic target, (iii) the nucleotide triplet in positions -5 to -3 is identical to the nucleotide triplet which is present in positions -5 to -3 of said genomic target and (iv) the nucleotide triplet in positions +3 to +5 is identical to the reverse complementary sequence of the nucleotide triplet which is present in positions -5 to -3 of said genomic target,
(h₁) combining in a single variant, the mutation(s) in positions 26 to 40 and 44 to 77 of two variants from step (e₁) and step (f₁), to obtain a novel homodimeric I-*Cre*I variant which cleaves a sequence wherein (i) the nucleotide triplet in positions +3 to +5 is identical to the nucleotide triplet which is present in positions +3 to +5 of said genomic target, (ii) the nucleotide triplet in positions -5 to - 3 is identical to the reverse complementary sequence of the nucleotide triplet which is present in positions +3 to +5 of said genomic target, (iii) the nucleotide triplet in positions +8 to +10 of the I-*Cre*I site has been replaced with the nucleotide triplet which is present in positions +8 to +10 of said genomic target and (iv) the nucleotide triplet in positions -10 to -8 is identical to the reverse complementary sequence of the nucleotide triplet in positions +8 to +10 of said genomic target,
(i₁) introducing in the variants from step (g₁) and/or (h₁), at least one mutation in the final C-terminal loop, preferably a substitution in position 138, 139, 142 or 143 of I-*Cre*I, as defined above,
(j₁) combining the variants obtained in steps (g₁), (h₁) and/or (i₁) to form heterodimers, and
(k₁) selecting and/or screening the heterodimers from step (j₁) which are able to cleave said genomic DNA target situated in a gene of interest.

Alternatively, the I-*Cre*I variant according to the invention may be obtained by a second method for engineering I*-Cre*I variants able to cleave a genomic DNA target sequence from a gene of interest, comprising at least the steps of:
(a₂) constructing a first series of I-*Cre*I variants having at least one substitution in a first functional subdomain of the LAGLIDADG core domain situated from positions 26 to 40 of I-*Cre*I and one mutation in the final C-terminal loop, preferably a substitution in position 138, 139, 142 or 143 of I-*Cre*I, as defined above,
(b₂) constructing a second series of I-*Cre*I variants having at least one substitution in a second functional subdomain of the LAGLIDADG core domain situated from positions 44 to 77 of I-*Cre*I and one mutation in the final C-terminal loop, preferably a substitution in position 138, 139, 142 or 143 of I-*Cre*I, as defined above, with the proviso that at least one of the two series of I-*Cre*I variants comprise at least one mutation in the final C-terminal loop,
(c₂) selecting and/or screening the variants from the first series of step (a₂) which are able to cleave a mutant I-*Cre*I site wherein (i) the nucleotide triplet in positions -10 to -8 and eventually at least one of the nucleotide doublet(s) in positions -12 to -11, -7 to -6 and/or -2 to -1 of the I-*Cre*I site have been replaced, respectively with the nucleotide triplet which is present in positions -10 to -8 and the nucleotide doublet which is present in positions -12 to -11, -7 to -6 and/or -2 to -1 of said genomic target (ii) the nucleotide triplet in positions +8 to +10 and eventually at least one of the nucleotide doublet(s) in positions +1 to +2, +6 to +7, and/or +11 to +12 have been replaced with the reverse complementary sequence of respectively, the nucleotide triplet which is present in positions -10 to -8 and the nucleotide doublet which is present in positions -12 to -11, -7 to -6 and/or -2 to -1 of said genomic target,
(d₂) selecting and/or screening the variants from the second series of step (b₂) which are able to cleave a mutant I-*Cre*I site wherein (i) the nucleotide triplet in positions -5 to -3 and eventually at least one of the nucleotide doublet(s) in positions -12 to -11, -7 to -6 and/or -2 to -1 of the I-*Cre*I site have been replaced respectively with the nucleotide triplet which is present in positions -5 to -3 and the nucleotide doublet which is present in positions -12 to -11, -7 to -6 and/or -2 to -1 of said genomic target and (ii) the nucleotide triplet in positions +3 to +5 and eventually at least one of the nucleotide doublet(s) in positions +1 to +2, +6 to +7, and/or +11 to +12 have been replaced with the reverse complementary sequence of respectively the nucleotide triplet which is present in positions -5 to -3 and the nucleotide doublet which is present in positions -12 to -11, -7 to -6 and/or -2 to -1 of said genomic target, with the proviso that at least one of the two mutant I-*Cre*I sites in step (c) and (d) have mutation(s) in at least one of the nucleotide doublet(s) in position -12 to -11, -7 to -6 and/or -2 to -1 and at least one of the corresponding nucleotide doublet (s) in positions +1 to +2, +6 to +7, and/or +11 to +12 of the I-*Cre*I site,
(e₂) selecting and/or screening the variants from the first series of step (a₂) which are able to cleave a mutant I-*Cre*I site wherein (i) the nucleotide triplet in positions +8 to +10, and eventually at least one of the nucleotide doublet(s) in positions +1 to +2, +6 to +7, and/or +11 to +12 of the I-*Cre*I site have been replaced respectively with the nucleotide triplet which is present in positions +8 to +10, and the nucleotide doublet which is present in positions +1 to +2, +6 to +7, and/or +11 to +12 of said genomic target and (ii) the nucleotide triplet in positions -10 to -8 and eventually the nucleotide doublet(s) in positions -12 to -11, -7 to -6 and/or -2 to -1 have been replaced with the reverse complementary sequence of respectively the nucleotide triplet which is present in positions +8 to +10, and the nucleotide doublet which is present in positions +1 to +2, +6 to +7, and/or +11 to +12 of said genomic target,
(f₂) selecting and/or screening the variants from the second series of step (b₂) which are able to cleave a mutant I*-Cre*I site wherein (i) the nucleotide triplet in positions +3 to +5, and eventually the nucleotide doublet(s) in positions +1 to +2, +6 to +7, and/or +11 to +12 of the I-*Cre*I site, have been replaced respectively with the nucleotide triplet which is present in positions +3 to +5, and the nucleotide doublet(s) which is present in positions +1 to +2, +6 to +7, and/or +11 to +12 of said genomic target and (ii) the nucleotide triplet in positions -5 to -3, and eventually the nucleotide doublet(s) in positions -12 to -11, -7 to -6 and/or -2 to -1 have been replaced with the reverse complementary sequence of, respectively the nucleotide triplet which is present in positions +3 to +5, and the nucleotide doublet(s) in positions +1 to +2, +6 to +7, and/or +11 to +12 of said genomic target, with the proviso that at least one of the two mutant I-*Cre*I sites in step (e) and (f) have mutation(s) in at least one of the nucleotide doublet(s) in positions +1 to +2, +6 to +7, and/or +11 to +12 and at least one of the corresponding nucleotide doublet (s) in positions in positions -12 to -11, -7 to -6 and/or -2 to -1 of the I-*Cre*I site,
(g₂) combining in a single variant, the mutation(s) in positions 26 to 40, 44 to 77 and in the final C-terminal loop of two variants from step (c₂) and step (d₂), to obtain a novel homodimeric I-*Cre*I variant which cleaves a sequence wherein (i) the nucleotide triplet in positions -10 to -8 and the nucleotide doublet(s) in positions -12 to -11, -7 to -6 and/or -2 to -1 are identical, respectively to the nucleotide triplet which is present in positions -10 to -8, and the nucleotide doublet(s) in positions -12 to -11, -7 to -6 and/or -2 to -1 of said genomic target, (ii) the nucleotide triplet in positions +8 to +10 and the nucleotide doublet(s) in positions +1 to +2, +6 to +7, and/or +11 to +12 are identical to the reverse complementary sequence of, respectively the nucleotide triplet which is present in positions -10 to -8 and the nucleotide doublet(s) in positions -12 to -11, -7 to -6 and/or -2 to -1 of said genomic target, (iii) the nucleotide triplet in positions -5 to -3 is identical to the nucleotide triplet which is present in positions -5 to -3 of said genomic target and (iv) the nucleotide triplet in positions +3 to +5 is identical to the reverse complementary sequence of the nucleotide triplet which is present in positions -5 to -3 of said genomic target,
(h₂) combining in a single variant, the mutation(s) in positions 26 to 40, 44 to 77 and in the final C-terminal loop of two variants from step (e₂) and step (f₂), to obtain a novel homodimeric I-*Cre*I variant which cleaves a sequence wherein (i) the nucleotide triplet in positions +3 to +5 and the nucleotide doublet(s) in positions +1 to +2, +6 to +7, and/or +11 to +12 are identical, respectively to the nucleotide triplet which is present in positions +3 to +5 and the nucleotide doublet(s) in positions +1 to +2, +6 to +7, and/or +11 to +12 of said genomic target, (ii) the nucleotide triplet in positions -5 to -3 and the nucleotide doublet(s) in positions -12 to -11, -7 to -6 and/or -2 to -1 are identical to the reverse complementary sequence of, respectively the nucleotide triplet which is present in positions +3 to +5 and the nucleotide doublet(s) present in positions +1 to +2, +6 to +7, and/or +11 to +12 of said genomic target, (iii) the nucleotide triplet in positions +8 to +10 of the I-*Cre*I site has been replaced with the nucleotide triplet which is present in positions +8 to +10 of said genomic target and (iv) the nucleotide triplet in positions -10 to -8 is identical to the reverse complementary sequence of the nucleotide triplet in positions +8 to +10 of said genomic target,
(i₂) combining the variants obtained in steps (g₂) and (h₂) to form heterodimers, and
(j₂) selecting and/or screening the heterodimers from step (i₂) which are able to cleave said genomic DNA target situated in a gene of interest.

According to yet another alternative, the I-*Cre*I variant of the invention may be obtained by a third method for engineering I-*Cre*I variants able to cleave a genomic DNA target sequence from a gene of interest, comprising at least the steps of:
(a₃) constructing a first series of variants having at least one substitution in a first functional subdomain of the LAGLIDADG core domain situated from positions 26 to 40 of I-*Cre*I,
(b₃) constructing a second series of I-*Cre*I variants having at least one substitution in a second functional subdomain of the LAGLIDADG core domain situated from positions 44 to 77 of I-*Cre*I,
(c₃) constructing a third series of variants having at least one mutation in the final C-terminal loop, preferably a substitution in position 138, 139, 142 or 143 of I-*Cre*I, as defined above,
(d₃) selecting and/or screening the variants from the first series of step (a₃) which are able to cleave a mutant I-*Cre*I site wherein (i) the nucleotide triplet in positions -10 to -8 of the I-*Cre*I site has been replaced with the nucleotide triplet which is present in positions -10 to -8 of said genomic target and (ii) the nucleotide triplet in positions +8 to +10 has been replaced with the reverse complementary sequence of the nucleotide triplet which is present in positions -10 to -8 of said genomic target,
(e₃) selecting and/or screening the variants from the second series of step (b₃) which are able to cleave a mutant I-*Cre*I site wherein (i) the nucleotide triplet in positions -5 to -3 of the I*-Cre*I site has been replaced with the nucleotide triplet which is present in positions -5 to -3 of said genomic target and (ii) the nucleotide triplet in positions +3 to +5 has been replaced with the reverse complementary sequence of the nucleotide triplet which is present in positions -5 to -3 of said genomic target,
(f₃) selecting and/or screening the variants from the third series of step (c₃) which are able to cleave a mutant I-*Cre*I site wherein (i) the nucleotide doublet(s) in positions -12 to -11, -7 to -6 and/or -2 to -1 of the I-*Cre*I site has been replaced with the nucleotide triplet which is present in positions -12 to -11, -7 to -6 and/or -2 to -1, respectively, of said genomic target and (ii) the nucleotide doublet(s) in positions +1 to +2 ,+6 to +7, and/or +11 to +12 has been replaced with the reverse complementary sequence of the nucleotide triplet which is present in positions -2 to - 1, -7 to -6, and/or -12 to -11, respectively, of said genomic target,
(g₃) selecting and/or screening the variants from the first series of step (a₃) which are able to cleave a mutant I-*Cre*I site wherein (i) the nucleotide triplet in positions +8 to +10 of the I-*Cre*I site has been replaced with the nucleotide triplet which is present in positions +8 to +10 of said genomic target and (ii) the nucleotide triplet in positions -10 to -8 has been replaced with the reverse complementary sequence of the nucleotide triplet which is present in positions +8 to +10 of said genomic target,
(h₃) selecting and/or screening the variants from the second series of step (b₃) which are able to cleave a mutant I*-Cre*I site wherein (i) the nucleotide triplet in positions +3 to +5 of the I-*Cre*I site has been replaced with the nucleotide triplet which is present in positions +3 to +5 of said genomic target and (ii) the nucleotide triplet in positions -5 to -3 has been replaced with the reverse complementary sequence of the nucleotide triplet which is present in positions +3 to +5 of said genomic target,
(i₃) selecting and/or screening the variants from the third series of step (c₃) which are able to cleave a mutant I*-Cre*I site wherein (i) the nucleotide doublet(s) in positions +1 to +2, +6 to +7, and/or +11 to +12 of the I*-Cre*I site has been replaced with the nucleotide doublet(s) which is present in positions +1 to +2, +6 to +7, and/or +11 to +12, respectively, of said genomic target and (ii) the nucleotide doublet(s) in positions -12 to -11, -7 to -6 and/or -2 to -1 has been replaced with the reverse complementary sequence of the nucleotide doublet which is present in positions +11 to +12, +6 to +7, and/or +1 to +2, respectively, of said genomic target,
(j₃) combining in a single variant, the mutation(s) in positions 26 to 40, 44 to 77 and in the final C-terminal loop of three variants from step (d₃) (e₃) and (f₃), to obtain a novel homodimeric I-*Cre*I variant which cleaves a sequence wherein (i) the nucleotide triplet in positions -10 to -8 is identical to the nucleotide triplet which is present in positions -10 to -8 of said genomic target, (ii) the nucleotide triplet in positions +8 to +10 is identical to the reverse complementary sequence of the nucleotide triplet which is present in positions -10'to -8 of said genomic target, (iii) the nucleotide triplet in positions -5 to -3 is identical to the nucleotide triplet which is present in positions -5 to -3 of said genomic target and (iv) the nucleotide triplet in positions +3 to +5 is identical to the reverse complementary sequence of the nucleotide triplet which is present in positions -5 to -3 of said genomic target, (v) the nucleotide doublet(s) in positions -12 to -11, -7 to -6 and/or -2 to -1 is identical to the nucleotide triplet which is present in positions -12 to -11, -7 to -6 and/or -2 to -1, respectively, -of said genomic target, (vi) the nucleotide doublet(s) in positions +1 to +2 ,+6 to +7, and/or +11 to +12 is identical to the reverse complementary sequence of the nucleotide doublet(s) which is present in positions -2 to -1, -7 to -6, and/or -12 to - 11, respectively, of said genomic target,
(k₃) combining in a single variant, the mutation(s) in positions 26 to 40, 44 to 77 and in the final C-terminal loop of three variants from step (g₃) (h₃) and (i₃), to obtain a novel homodimeric I-*Cre*I variant which cleaves a sequence wherein (i) the nucleotide triplet in positions +3 to +5 is identical to the nucleotide triplet which is present in positions +3 to +5 of said genomic target, (ii) the nucleotide triplet in positions -5 to -3 is identical to the reverse complementary sequence of the nucleotide triplet which is present in positions +3 to +5 of said genomic target, (iii) the nucleotide triplet in positions +8 to +10 of the I-*Cre*I site has been replaced with the nucleotide triplet which is present in positions +8 to +10 of said genomic target and (iv) the nucleotide triplet in positions -10 to -8 is identical to the reverse complementary sequence of the nucleotide triplet in positions +8 to +10 of said genomic target, (v) the nucleotide doublet(s) in positions +1 to +2 ,+6 to +7, and/or +11 to +12 is identical to the nucleotide doublet(s) which is present in positions +1 to +2 ,+6 to +7, and/or +11 to +12, respectively of said genomic target, (vi) the nucleotide doublet(s) in positions -12 to -11, -7 to -6 and/or -2 to -1 is identical to the reverse complementary sequence of the nucleotide doublet(s) which is present in positions +11 to +12, +6 to +7, and/or +1 to +2, respectively of said genomic target,
(l₃) combining the variants obtained in steps (j₃) and (k₃) to form heterodimers, and
(m₁) selecting and/or screening the heterodimers from step (l₃) which are able to cleave said genomic DNA target situated in a gene of interest.

The steps (a₁), (a₂), (b₁), (b₂), (a₃), (b₃), (c₃), (g₁), (g₂), (h₁), (h₂), (i₁), (j₃), and (k₃) may comprise the introduction of additional mutations in order to improve the binding and/or cleavage properties of the mutants, particularly at other positions contacting the DNA target sequence or interacting directly or indirectly with said DNA target. These steps may be performed by generating a combinatorial library as described in the International PCT Application WO 2004/067736, Arnould et al., J. Mol. Biol., 2006, 355, 443-458 and Smith et al., Nucleic Acids Research, Epub 27 November 2006.

Steps (g₁), (g₂), (h₁), (h₂), (i₁), (j₃) and (k₃), may further comprise the introduction of random mutations on the whole variant or in a part of the variant, in particular the C-terminal half of the variant (positions 80 to 163). This may be performed by generating random mutagenesis libraries on a pool of variants, according to standard mutagenesis methods which are well-known in the art and commercially available.

Step (i₁) may also comprise the selection and/or screening of the homodimers which are able to cleave a sequence wherein the nucleotide doublet in positions +1 to +2, +6 to +7 and/or +11 to +12 is identical to the nucleotide doublet which is present in positions +1 to +2, +6 to +7 and/or +11 to +12, respectively of said genomic target, and the nucleotide doublet in positions -12 to -11, -7 to -6, and/or -2 to -1 is identical to the reverse complementary sequence of the nucleotide triplet which is present in positions +11 to +12, +6 to +7, and/or +1 to +2, respectively of said genomic target.

The (intramolecular) combination of mutations in steps (g₁), (g₂), (h₁), (h₂), (j₃) and (k₃) may be performed by amplifying overlapping fragments comprising each of the two subdomains, according to well-known overlapping PCR techniques, as described for example in Smith et al., Nucleic Acids Res., Epub 27 November 2006.

The (intermolecular) combination of the variants in step (j₁), (i₂) and (l₃) is performed by co-expressing one variant from step (g₁), (g₂) or (i₁), (j₃) with one variant from step (h₁), (h₂) or (i₁), (k₃), respectively, so as to allow the formation of heterodimers. For example, host cells may be modified by one or two recombinant expression vector(s) encoding said variant(s). The cells are then cultured under conditions allowing the expression of the variant(s), so that heterodimers are formed in the host cells, as described previously in Arnould et al., J. Mol. Biol., 2006, 355, 443-458; International PCT Applications WO 2006/097853, WO 2006/097854 and WO 2006/097784; Smith et al., Nucleic Acids Res., Epub 27 November 2006.

The selection and/or screening steps may be performed by using a cleavage assay *in vitro* or *in vivo,* as defined above. Preferably, it is performed *in vivo,* under conditions where the double-strand break in the mutated DNA target sequence which is generated by said variant leads to the activation of a positive selection marker or a reporter gene, or the inactivation of a negative selection marker or a reporter gene, by recombination-mediated repair of said DNA double-strand break, as defined above.

The subject-matter of the present invention is also the use of at least one meganuclease, as defined above, as a scaffold for making other meganucleases. For example other rounds of mutagenesis and selection/screening can be performed on the variant, for the purpose of making novel homing endonucleases.

The subject-matter of the present invention is also a method for decreasing the toxicity of a parent LAGLIDADG homing endonuclease, comprising: the mutation of at least one amino acid of the final C-terminal loop of said parent LAGLIDADG homing endonuclease.

According to an advantageous embodiment of said method the parent endonuclease is I-*Cre*I or a functional variant thereof. Preferably, the K139 and/or T143 residues are mutated. More preferably K139 is mutated in an hydrophobic amino acid such as a methionine (K139M) and/or T143 is mutated in a small amino acid such as a glycine (T143G).

The polynucleotide fragments having the sequence of the targeting DNA construct or the sequence encoding the meganuclease variant or single-chain meganuclease derivative as defined in the present invention, may be prepared by any method known by the man skilled in the art. For example, they are amplified from a DNA template, by polymerase chain reaction with specific primers. Preferably the codons of the cDNAs encoding the megaunclease variant or single-chain meganuclease derivative are chosen to favour the expression of said proteins in the desired expression system.

The recombinant vector comprising said polynucleotides may be obtained and introduced in a host cell by the well-known recombinant DNA and genetic engineering techniques.

The meganuclease variant or single-chain meganuclease derivative as defined in the present the invention are produced by expressing the polypeptide(s) as defined above; preferably said polypeptide(s) are expressed or co-expressed (in the case of the variant only) in a host cell or a transgenic animal/plant modified by one expression vector or two expression vectors (in the case of the variant only), under conditions suitable for the expression or co-expression of the polypeptide(s), and the meganuclease variant or single-chain meganuclease derivative is recovered from the host cell culture or from the transgenic animal/plant.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

In addition to the preceding features, the invention further comprises other features which will emerge from the description which follows, which refers to examples illustrating I-CreI meganuclease variants and their uses according to the invention, as well as to the appended drawings in which:
- figure 1 represents the superposition of the Cα ribbon representation of the I-CreI and I-CreI-DNA structures. DNA has been omitted for clarity.
- figure 2 represents the sequence alignment of the C-terminal region from members of the I-CreI family (Lucas et al., Nucleic Acids Res., 2001, 29, 960-969). The position of the mutated residues in the SKTRKTT motif is indicated with a grey triangle (http://espript.ibcp.fr/ESPript/cgi-bin/ESPript.cgi).
- figure 3 represents a detailed view of S138, K139, K142 and T143 contacts with the DNA backbone (a) and the comparison of the positions of S138, K139, K142 and T143 between the bound and unbound DNA structures (b).
- figure 4 illustrates the biophysical characterization of the I-CreI C-terminal region mutants. a) Circular dichroism thermal denaturation. b) Monodimensional H-H NMR spectra.
- figure 5 illustrates dimer formation by the I-*Cre*I C-terminal region mutants, measured by analytical ultracentrifugation. Sedimentation velocity distribution of the *I-CreI* proteins (1 mg/ml in PBS buffer) at 42,000 rpm and 20°C. Inset, sedimentation equilibrium gradient of I-*Cre*I proteins (4 mg/ml in PBS buffer) at 11,000 rpm and 20°C. Open circles represent the experimental data, the two solid lines represent the theoretical gradients of a I-*Cre*I monomer (20,045) and dimer (41,000).
- figure 6 represents electrophoretic mobility shift assays of the C-terminal truncated, double and single mutants in the presence of Mg²⁺ and Ca²⁺.
- figure 7 is a summary of the gel *in vitro* cleavage assay of the C-terminal truncated, double and single mutants.
- figure 8 illustrates the *in vivo* cleavage assay used for profiling the single mutants and the 10NNN_P DNA target cleavage profile of the single mutants. a) Yeast screening assay principle. A strain harboring the expression vector encoding a single mutant is mated with a strain harboring a reporter plasmid. In the reporter plasmid, a LacZ reporter gene is interrupted with an insert containing one of the target sites of interest, flanked by two direct repeats. Upon mating, the meganuclease (grey oval) generates a double-strand break at the site of interest, allowing restoration of a functional LacZ gene by single-strand annealing (SSA) between the two flanking direct repeats. The functional LacZ gene is visualized by a blue staining. b) DNA targets. The C1221 target (top) is a palindromic target cleaved by I-*Cre*I. All targets used in this study are palindromic targets derived from C1221 by substitution of six nucleotides in ±8, ±9 and ±10 (SEQ ID NO: 1 and 10 to 16). A few examples are shown (bottom). The 10GGG_P target differs from the C1221 target by the GGG triplet in -10, -9, -8 and CCC in +8, +9 and +10. c) Mutant target profiles. Each mutant was profiled in yeast on a series of 64 palindromic targets (10NNN_P). An example of cleavage activity in yeast for a single mutant (K139M) compared to I-*Cre*I D75N is presented. Blue staining indicates cleavage. Additionally a representation of the 10NNN_P cleavage profile of all single mutants compared to I-*Cre*I D75N and I-*Cre*I. Grey levels reflect the intensity of the signal. I-*Cre*I is toxic in yeast and profiles have been established at 30 °C instead of 37° C. All other mutants were studied at 37°C.
- figure 9 illustrates the 5NNN_P DNA target cleavage profile of the single mutants. The targets (64) are palindromic targets with variations in positions ± 3 to 5).
- figure 10 illustrates the 2NN DNA target cleavage profile of the single mutants. The targets (16×16) are non-palindromic targets with variations in positions ± 1 to 2.
- figure 11 illustrates the 12NN_P DNA target (A) and 7NN_P DNA target (B) cleavage profiles of the single mutants. The targets in A (16) and B (16) are palindromic targets with variations in positions ± 11 to 12 and ± 6 to 7, respectively.

### Example 1: Structural differences between the bound and unbound I-CreI DNA structures

### 1) Materials and Methods

### a) Protein expression,_purification and crystallization

Protein expression and purification was performed as in (Arnould et al., J. Mol. Biol., 2006, 355, 443-458). An initial screening for I-*Cre*I crystallization conditions was performed in 96 well plates by vapour-diffusion methods using the Hampton crystal screening using drops containing 1 µl protein solution (7 mg/ml in 20 mM HEPES, pH 7.5) and 1 µl precipitant solution equilibrated against 50 µl of reservoir solution at 20° C. Crystals were obtained under several conditions (Crystal Screen 1 conditions 10, 22, 33, 40, 41 and Crystal Screen 2 condition 32). Crystal was made by hanging-drop vapour-diffusion methods using VDX plates; optimization experiments led to the following conditions for crystallization: 1 µl protein at 7 mg/ml in 20 mM HEPES pH 7.5 and 1 µl precipitating buffer containing 20 % PEG 4000, 0.1 M HEPES pH 7.5, 10 % Iso-propanol, 10 % Ethylene glycol and 0.01 M Magnesium acetate equilibrated against 500 µl precipitating buffer at 20° C. Rod-shaped crystals grown in 4-8 days and were directly collected and frozen in liquid nitrogen.

### b) Data collection, structure solution, model building and refinement

All data were collected at cryogenic temperatures using synchrotron radiation at 100K. I-*Cre*I crystals were mounted and cryoprotected. The data sets were collected using synchrotron radiation at the ID14-4 beamline at the ESRF (Grenoble), and at the PX beamline at the SLS (Villigen). Diffraction data were recorded on an ADSC-Q4 or Mar225 CCD detectors depending on the beamline. Processing and scaling were accomplished with HKL2000 (Otwinowski, Z. and Minor, W.: Processing of X-ray Diffraction Data Collected in Oscillation Mode, In Methods in Enzymology, 1997, Academic Press, New York*).* The structure was solved using the molecular replacement method as implemented in the program MOLREP (Vagin, A. and Teplyakov, A. Acta Crystallogr. D Biol. Crystallogr., 2000, 56 Pt 12, 1622-1624).

### 2) Results

The structure of the I-CreI was solved by molecular replacement and refinement to 2.0Å resolution. The best data set (Table I) was collected using a Δϕ =1° and a wavelength of 0.97 Å. Statistics for the crystallographic data are summarized in Table I. The search model was based on a poly-alanine backbone derived from the PDB 1gz9 found in the Protein Data Bank. The coordinates from the DNA were deleted in the search model. A refined 2Fo-Fc map showed clear and contiguous electron density for the protein backbone and for many of the side-chains. ARP/wARP and REFMAC5 were applied for automatic model building and refinement to 2.0 Å (Table I).

**Table I: Data collection and refinement statistics**

| ***Data Collection*** | |
|---|---|
| Space group | P4₃ |
| *Number of crystals* | 1 |
| *Temperature (K)* | 100 |
| Wavelength (Å) | 0.97 |
| Cell dimensions (Å, °), | a=b=69.088, c=93.040 αβγ=90° |
| No. mol ASU | 2 |
| Data collection environment, beamline | ADSC-Q4, ID14-4 ESRF |
| Completeness (%) | 93.2 |
| Multiplicity | 5.3 |
| Rsym(%) | 6 |

| ***Refinement*** | |
|---|---|
| No Reflections | 25943 |
| Resolution range (Å) | 34.54-2.00 |
| R-factor / R-free (%) | 18/23 |
| No protein atoms (Average B, Å²)^{c} | |
| No water molecules (Average B, Å²)^{c} | |
| r.m.s bond length (Å) | 0.029 |
| r.m.s. bond angle (°) | 2.137 |
| Ramachandran plot outliers (number)^{d} | 0 |

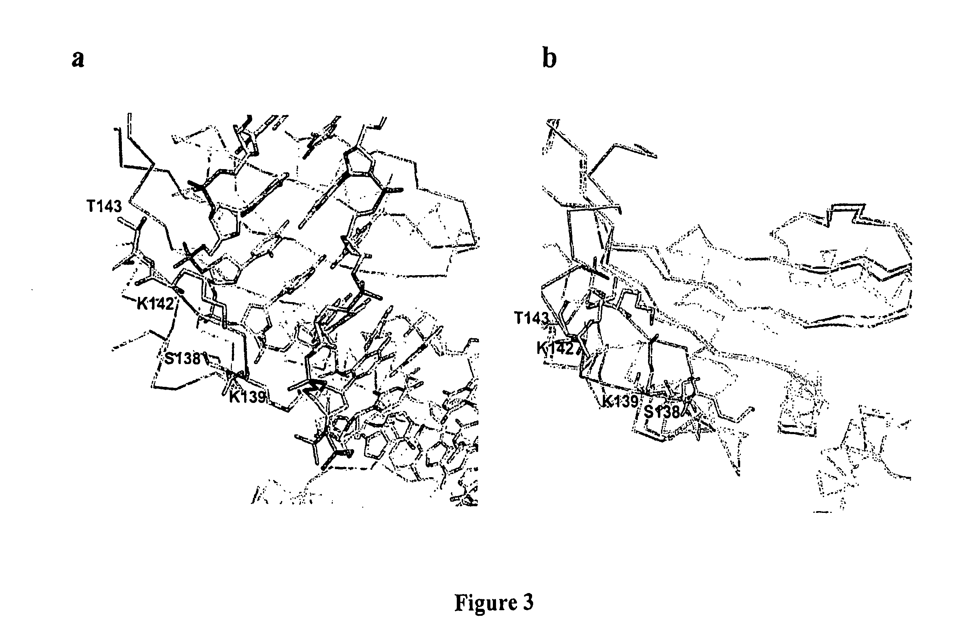

The dimer without DNA allowed the observation of the protein conformational changes upon DNA binding after comparison with the protein-DNA complex (PDB code 1gz9) (Figure 1). The most striking differences are in the C-terminal region conformation. Whereas in the DNA bound structure the C-helix and the C-loop are aligned with the DNA, in the unbound structure both elements are located on top of the cavity where the DNA binds, suggesting that the loop and the C-helix could work as a lock opening and closing the DNA binding groove. This region was not observed in a previous structure of I-*Cre*I with only one monomer in the asymmetric unit (Heath et al., Nat Struct Biol, 1997, 4, 468-476). Besides, the C-terminal domain of I-*Cre*I is well conserved among homodimeric proteins from the LAGLIDADG family (Lucas et al., Nucleic Acids Res., 2001, 29, 960-969) indicating its important role in this meganuclease group working mechanism (Figure 2). A detailed view of the protein-DNA interactions in the C-terminal area showed that Ser138, Lys139, Lys142 and Thr143 at the SKTRKTT motif are involved in hydrogen bonds with the DNA backbone (Figure 3a). The position of these residues is completely different in the unbound DNA state (Figure 3b), indicating that a conformational change is needed to bind the nucleic acid. Although these interactions were described before (Chevalier et al., J. Mol. Biol., 2003, 329, 253-269) and the amino acids are conserved, there is no information about their role during meganuclease action.

### Example 2: Biophysical analysis

### 1) Materials and Methods

### a) Construction of the I-CreI mutants

The I-*Cre*I deletion mutants (Δ1 and Δ2) were amplified by PCR on the wild-type I-*Cre*I (I-*Cre*I D75) cDNA template, with the forward primer 5' gatataccatggccaataccaaatataac 3' (SEQ ID NO: 18) for both mutants and the reverse primer ICreI deltaCter-R: 5' ttatcagtcggccgcatcgttcagagctgcaatctgatccacccagg 3' (SEQ ID NO: 19) for the Δ1 mutant or Creh2: 5' gagtgcggccgcagtggttttacgcgtcttagaatcg 3' (SEQ ID NO: 20) for the Δ2 mutant.

The I-*Cre*I single and double mutants were amplified by round-the-world PCR with a Quickchange® kit (STRATAGENE # 200518), appropriate mutagenizing oligos and the wild-type I-*Cre*I (I-CreI D75) cDNA as template.

### b) Circular Dichroism thermal analysis

Data were acquired with a Jasco 810 model dichrograph, previously calibrated with *d*-10-camphorsulphonic acid, and equipped with a Jasco Peltier thermoelectric temperature controller CDF-426S model. Experiments were performed in PBS at 1° C/min intervals. The protein concentration was 10 µM. The ellipticity at 222 nm was followed from 5 to 95 °C in a 2 mm Hellma 110-QS cell.

### c) Analytical utracentrifugation

Sedimentation equilibrium experiments were performed at 20 °C in an Optima XL-A (Beckman-Coulter) analytical ultracentrifuge equipped with UV-visible optics, using an An50Ti rotor, with 3,mm double sector centerpieces of Epon charcoal. Protein concentration was 200 µM in PBS buffer. Short column (23 µl), low speed sedimentation equilibrium was performed at three successive speeds (11,000, 13,000, and 15,000 rpm), the system was assumed to be at equilibrium when successive scans overlaid and the equilibrium scans were obtained at wavelength of 280 nm. The base-line signal was measured after high speed centrifugation (5 h at 42,000 rpm). Whole-cell apparent molecular weight of the protein was obtained using the program EQASSOC *(*Minton, A.P., In: Modern Analytical Ultracentrifugation, 1994, Birkhauser Boston, Inc., Cambridge, MA*).* The partial specific volume of I-*Cre*I was 0.7436 ml/g at 20°C, calculated from the amino acid composition with the program SEDNTERP (retrieved from the RASMB server; Laue, T.M.S., B.D., Ridgeway, T.M., Pelletier, S.L., In: Computer-aided interpretation of analytical sedimentation data for proteins, 1992, Royal Society of Chemistry, Cambridge, UK*).* The sedimentation velocity experiment was carried out in an XL-A analytical ultracentrifuge (Beckman-Coulter Inc.) at 42,000 rpm and 20° C, using an An50Ti rotor and 1.2mm double-sector centerpieces. Absorbance scans were taken at 280 nm. The protein concentration was 50 µM in PBS. The sedimentation coefficients were calculated by continuous distribution c(s) Lamm equation model (Schuck, P., Biophys. J., 2000, 78, 1606-1619) as implemented in the SEDFIT program. These experimental sedimentation values were corrected to standard conditions to get the corresponding *s*_{20,w} values using the SEDNTERP program *(*Laue, T.M.S., B.D., Ridgeway, T.M., Pelletier, S.L., In: Computer-aided interpretation of analytical sedimentation data for proteins, 1992, Royal Society of Chemistry, Cambridge, UK*).* Further hydrodynamic analysis (i.e. calculation of frictional coefficient ratio) was performed with the SEDFIT program to obtain de c(M) distribution (Schuck, P., Biophys. J., 2000, 78, 1606-1619).

### d) NMR data acquisition

NMR spectra were recorded at 25°C in a Bruker AVANCE 600 spectrometer equipped with a cryoprobe. Protein samples were 500 µM in PBS buffer (137 mM NaCl, 10 mM Na₂HPO₄-2H₂0, 2.7 mM KCI, 2 mM KH₂PO₄, pH 7.4) plus 5 % ²H₂O. DSS (2,2-Dimethyl-2-silapentane-5-sulfonate sodium salt) was used as internal proton chemical shift reference.

### 2) Results

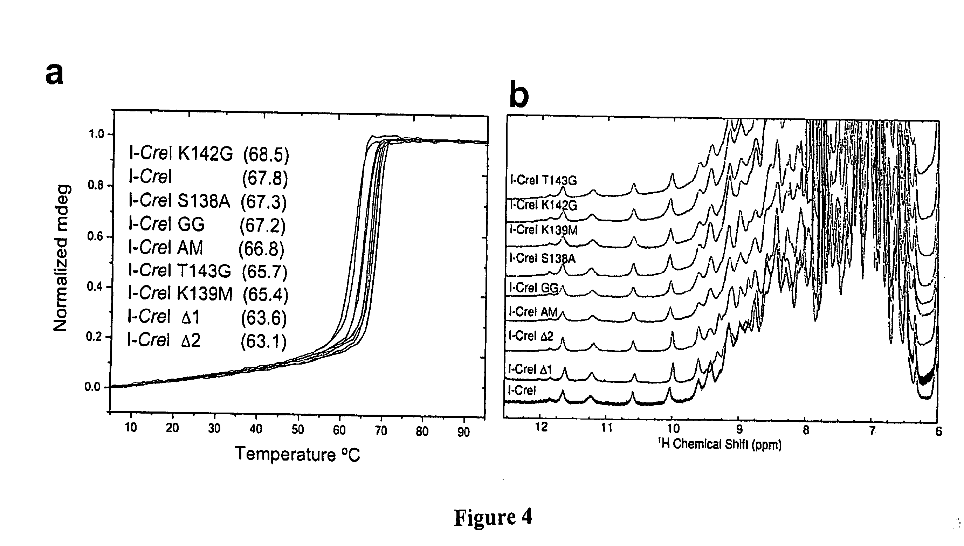

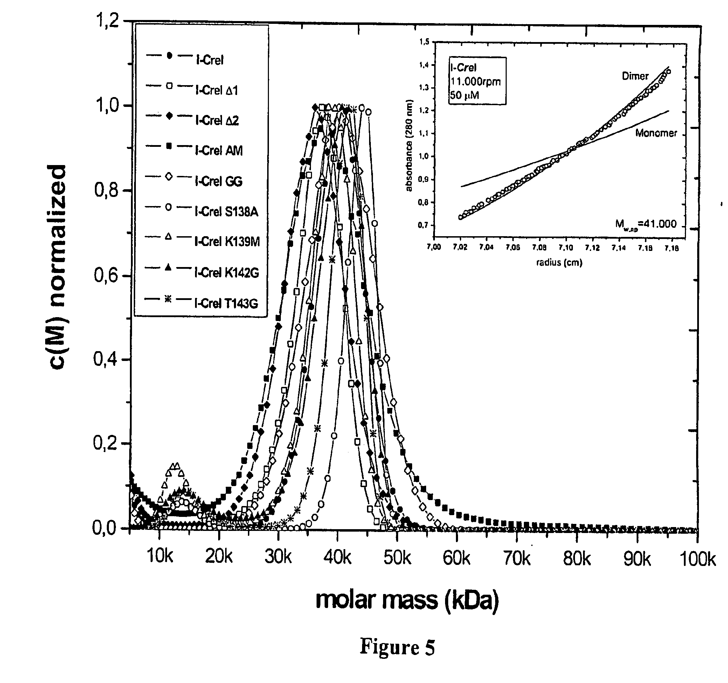

To unravel the role of the C-terminal domain of I-*Cre*I, a series a series of trimmed, double and single mutants were designed based on the structural differences between the bound and unbound DNA structures. The two truncated mutants were designed to clarify the role of the C-terminal region. I-*Cre*I Δ1 (amino acid number 1-137) lacked both the C-loop and the C-Helix whereas I-*Cre*I Δ2 (aminoacid number 1-144) contained the C-loop. Based on the contacts with the DNA backbone in the SKTRKTT motif, the double mutants *I-CreI* AM (S138A, K139M) and I-*Cre*I GG (K142G, T143G) were produced, as well as their single variants I-*Cre*I S138A, I-*Cre*I K139M, I-*Cre*I K142G, I-*Cre*I T143G. To demonstrate that the effect in meganuclease activity was due to the mutations, their effect in the protein stability, structure and oligomerization state, was studied. Thermal denaturation circular dichroism was performed to confirm that all the mutants were folded. Indeed, all the mutants displayed a sigmoidal curve similar to the wild type (Figure 4a) with different Tm depending on the mutation. In addition, monodimensional H-H NMR confirmed the thermal denaturation experiments, a well defined dispersion of peaks in the amide region demonstrated that all the mutants were folded (Figure 4b). It is well known that the I-*Cre*I family of meganucleases binds DNA as homodimers, therefore to analyze the oligomerization state of the mutants they were subjected to analytical ultracentrifugation. The experiment showed that all the mutants behaved as dimers independently of the mutation, with only little variations corresponding to their molecular weights (Figure 5).

Altogether these experiments indicate that the mutants are folded and conserve the I-*Cre*I scaffold involved in meganuclease activity.

### Example 3: DNA-binding activity the C-terminal mutants

### 1) Materials and Methods

### Band shift assay conditions

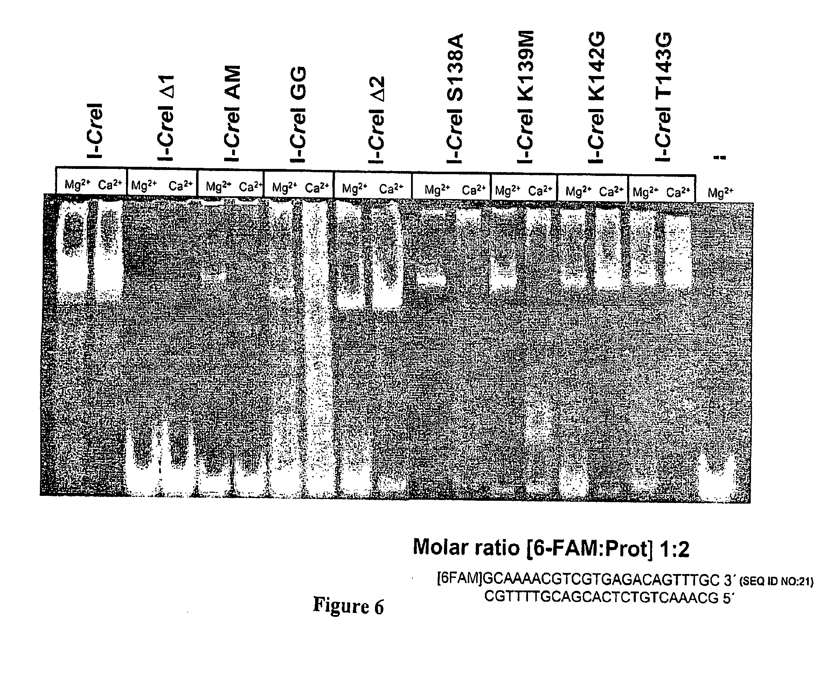

Band shift assays were performed in 10 mM Tris-HCl pH 8, 50 mM NaCl, 10 mM CaCl₂ or MgCl₂, 1 mM DTT incubated 1 h at room temperature using 5 µM (0.0793 µg/µl) 6-FAM duplex (SEQ ID NO: 21; see figure 6) and 20 µM (0.463 µg/µl) protein and electrophoresed in a 15 % Acrylamide-TBE gel.

### 2) Results

Electrophoretic mobility shift assays (EMSA) in the presence of Mg²⁺ and Ca²⁺ were used to analyze the behavior of the C-terminal mutants in DNA binding (Figure 6). Whereas the presence of Ca²⁺ allows DNA binding, Mg²⁺ is indispensable to bind and cleave DNA (Chevalier et al., Biochemistry, 2004, 43, 14015-14026). Even though the binding capability of I-*Cre*I was abolished in the Δ1 mutant, the Δ2 was able to bind the labeled DNA probe demonstrating that the C-loop is essential in DNA binding. In addition, binding was detected in the presence of both cations as in the wild type *I-CreI.*

On the other hand, both I-*Cre*I AM and I-*Cre*I GG double mutants were severely affected in their DNA binding properties independently of the cation present, indicating that Ser138, Lys139, Lys142 and Thr143 contacts with the DNA backbone are crucial to bind the nucleic acid. Therefore, these residues in the SKTRKTT motif constitute two new hot-spots essential for I-*Cre*I DNA binding.

To define the distinct properties of each site in the C-loop, the single mutants were assayed by EMSA in the same conditions. In contrast with the double mutants all the single ones were able to bind the labeled probe; however they displayed differences depending of the cation present in the assay. Whereas a clear dependence of Mg could be observed in the Ser138-Lys139 site, the single mutants in the Lys142-Thr143 site could bind DNA notwithstanding the cation present in the mobility assay.

Thus, the mutation of both residues in each site is needed to abolish DNA binding, indicating that a synergy between the two residues in each hot-spot is essential for DNA binding.

### Example 4: DNA-cleavage activity of the C-terminal mutants, in vitro

### 1) Materials and Methods

### In vitro cleavage assay conditions

Cleavage assays were performed at 37°C in 10 mM Tris-HCl (pH 8), 50 mM NaCl, 10 mM MgCl₂ (or CaCl₂) and 1 mM DTT. Concentrations were: 100 ng for the *Xmn*I linearized target substrate (pGEM-T Easy C1221 GTC) and 40-0.25ng dilutions for I-*Cre*I and helix mutant proteins, in 25 µl final volume reaction. The linearized target plasmid has 3 kb and after cleavage yields two smaller bands of 2 kb and 1 kb. Reactions were stopped after 1 hour by addition of 5 µl of 45 % Glycerol, 95 mM EDTA (pH 8), 1.5 % (w/v) SDS, 1.5 mg/ml Proteinase K and 0.048 % (w/v) Bromophenol blue (6x Buffer Stop), incubated at 37°C for 30 minutes and electrophoresed in a 1 % agarose gel. The fragments were quantified using SYBR Safe DNA gel staining (IN VITROGEN). Gels were analysed using the ImageJ software (http://rsb.info.nih.gov/ij/) to calculate the percentage of cleavage according to (2kb+1kb)/(3kb+2kb+1kb)*100 formula.

### 2) Results

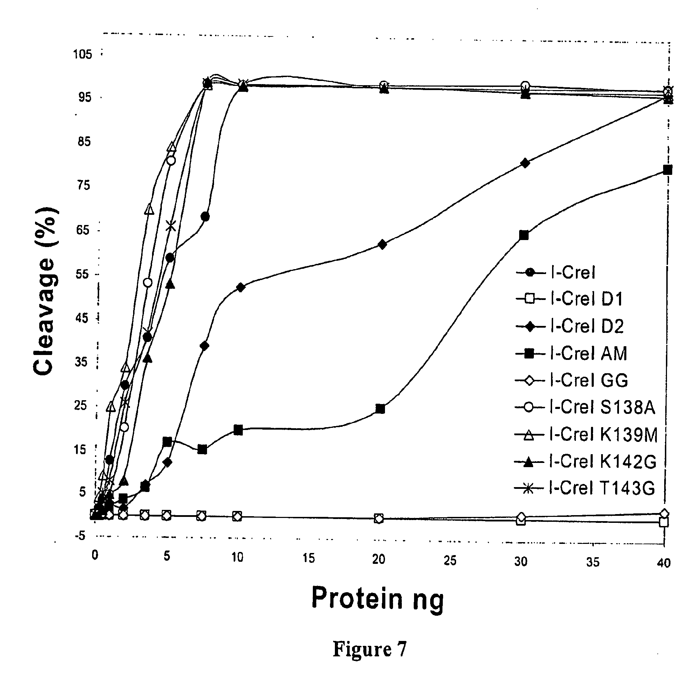

The analysis of the distinct mutants in the DNA binding assays has clear implications for DNA cleavage activity, consequently an examination of their cleavage properties on a wild type DNA sequence was carried out. Figure 7 displays a graph representing the percentage of cleavage against the amount of HE (Gels with raw data are available as supporting information). The mutants can be divided in two groups based on the comparison of their cleavage properties to the wild type HE; the first is composed of the truncated mutants I-CreI Δ1 and I-CreI Δ2 and the double mutants I-CreI AM and I-CreI GG which are , whereas the single mutants I-CreI S138A, I-CreI K139M, I-CreI K142G, I-CreI T143G form the second. Members the first group displayed a reduced cleavage activity when compared to the wild type I-Crel. Although I-CreI Δ1and I-CreI GG cleavage properties are completely abolished, I-CreI Δ2and I-CreI AM showed a reduced activity that is increased when higher HE amounts are used. However the cleavage properties of the single mutants that composed the second group are not only similar to the wild type, but enhanced in some cases (Figure 7).

These results indicate that the trimmed and double mutants whose DNA binding is abolished or severely affected do not cleave DNA or they need higher amounts of HE to cleave the plasmid. Noteworthy is the case of the I-Cre Δ2, the mutant that conserves the wild type amino acids in the C-loop but lacks the α6 helix, even though its cleavage activity is affected the activity profile is the more similar to the I-CreI wild type.

On the other hand the single mutants depict a slightly enhanced activity with respect to the wild type in all them. The activity assays confirm the DNA binding studies, indicating that the double mutants act in a concerted manner, however the effect of these mutations have implications not only in nucleic acid binding but also in DNA cleavage as we have shown.

### Example 5: DNA-cleavage activity of the C-terminal mutants, in vivo

### 1) Materials and Methods

The *in vivo* cleavage assay (Figure 8a) has been described previously in PCT Application WO 2004/067736; Epinat et al., Nucleic Acids Res., 2003, 31, 2952-2962; Chames et al., Nucleic Acids Res., 2005, 33, e178, and Arnould et al., J. Mol. Biol., 2006, 355, 443-458.

### a) Construction of target clones

The C1221 twenty-four bp target sequence (5'-tcaaaacgtcgtacgacgttttga-3': SEQ ID NO: 1) is a palindrome of a half-site of the natural I-*Cre*I target (5'-tcaaaacgtcgtgagacagtttgg-3': SEQ ID NO: 17). C1221 is cleaved as efficiently as the I-CreI natural target *in vitro* and *ex vivo* in both yeast and mammalian cells. The palindromic targets, derived from C1221, were cloned as previously described (Arnould et al., J. Mol. Biol., 2006, 355, 443-458) using the Gateway protocol (Invitrogen) into the reporter vectors: the yeast pFL39-ADH-LACURAZ and the mammalian vector pcDNA3.1-LACURAZ-ΔURA, both described previously (Epinat et al., Nucleic Acids Res., 2003, 31, 2952-2962) and containing a I-*Sce*I target site as control. Yeast reporter vectors were transformed into *S*. *cerevisiae* strain FYBL2-7B (*MAT a, urα3Δ851, trp1Δ63, leu2Δ1, lys2Δ202).*

### b) Screening in yeast

The protocol for screening homodimer mutants is as described previously (PCT Application WO 2004/067736; Epinat et al., Nucleic Acids Res., 2003, 31, 2952-2962; Chames et al., Nucleic Acids Res., 2005, 33, e178; Arnould et al., J. Mol. Biol., 2006, 355, 443-458).

### c) Mating of meganuclease expressing clones and screening in yeast

Mating was performed using a colony gridder (QpixII, Genetix). Mutants were gridded on nylon filters covering YPD plates, using a high gridding density (about 20 spots/cm²). A second gridding process was performed on the same filters to spot a second layer consisting of 64 or 75 different reporter-harboring yeast strains for each variant. Membranes were placed on solid agar YPD rich medium, and incubated at 30 °C for one night, to allow mating. Next, filters were transferred to synthetic medium, lacking leucine and tryptophan, with galactose (2 %) as a carbon source, and incubated for five days at 37°C (30°C for I-*CreI*), to select for diploids carrying the expression and target vectors. After 5 days, filters were placed on solid agarose medium with 0.02 % X-Gal in 0.5 M sodium phosphate buffer, pH 7.0, 0.1 % SDS, 6 % dimethyl formamide (DMF), 7 mM p-mercaptoethanol, 1 % agarose, and incubated at 37 °C, to monitor β-galactosidase activity. Results were analyzed by scanning and quantification was performed using appropriate software.

### 2) Results

To confirm the results *in vivo* cleavage assays were performed with all the mutants, as well as with the I-*Cre*I and I-*Cre*I D75N proteins, as described previously (Arnould et al., J. Mol. Biol., 2006, 355, 443-458).

None of the double mutants or truncated mutants, whose binding and cleavage activity were affected by the mutations *in vitro,* displayed activity on any of the targets. As an example the I-*Cre*I AM and I-*Cre*I GG are shown (Figure 8c upper panel). By contrast, all the single mutants showed high activity on the wild-type target (C1221).

### - 10NNN P target profiling (figure 8c)

All the single mutants showed high activity on the wild-type target (C1221), 10AAG_P and 10AAT_P. Lower levels of cleavage could also be observed with these four mutants with 10TCG_P and 10AAC_P. In addition the I-*Cre*I K139M mutant was also able to cleave seven additional targets (10AGT_P, 10GAG_P, 10GAA_P, 10GAT_P, 10CAG_P, 10CAA_P, 10CAT_P) as it can be observed in figure 8c. The profile of the I-CreI K139M mutant is very similar to I-*Cre*I (without its toxicity), while the three other single mutants are closer to I-*Cre*I D75N.

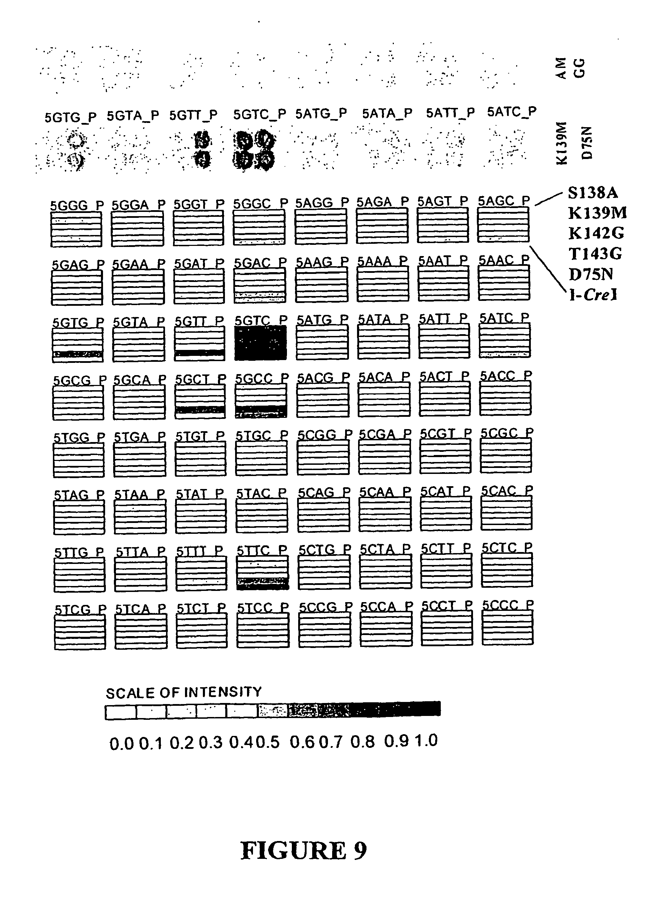

### - 5NNN P profiling (figure 9)

The profile of S138A and K139M is similar to the profile of I-*Cre*I D75N, whereas the profile of K142G and T143G is more restricted than the profile of I-*Cre*I D75N.

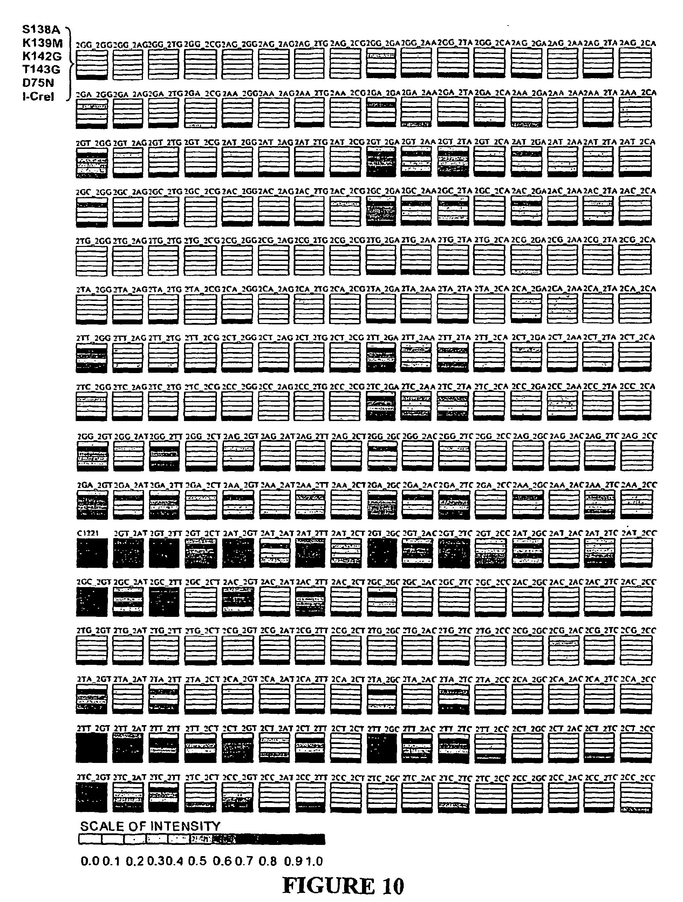

### - 2NN P profiling (figure 10)

The profile of K142G and S138A is more restricted than the profile of I-*Cre*I D75N. Compared to D75N, T143G and K139M cleave 6 and 10 additional targets, respectively, 6 of which are in common. In addition, at least 8 targets are cleaved more efficiently by K139M than by D75N. Five targets (2TT_2TG; 2TG_2TT, 2TA_2CT, 2TC_2TC, 2CT_2CT) are not cleaved by K139M; these targets are cleaved by D75N, although less efficiently than by *I-CreI.*

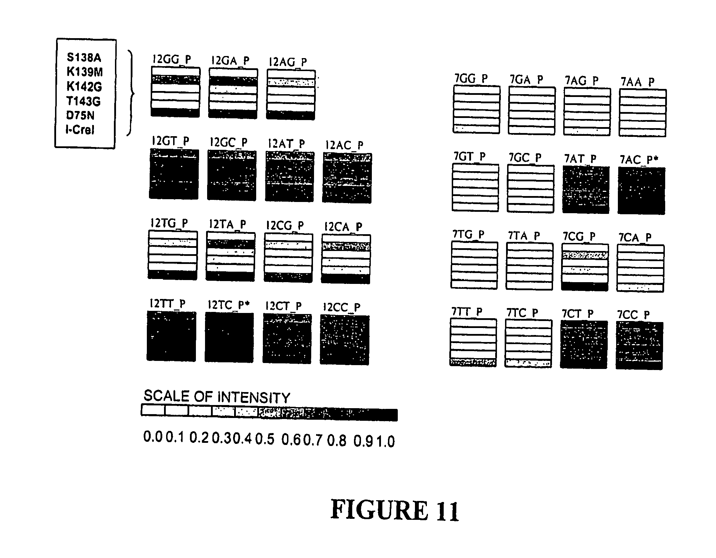

### - 12NN P profiling (Figure 11A)

The profile of K142G and S138A is more restricted than the profile of I-*Cre*I D75N, with the profile of S138A being more restricted than the profile of K142G.

The profile of T143G is similar to the profile of I-*Cre*I D75N.

The profile of K139M is similar to the profile of I-*Cre*I but without its toxicity; 7 additional targets are cleaved by K139M as compared to D75N.

### - 7NN P profiling (Figure 11B)

The profile of K142G and S138A is similar to the profile of I-*Cre*I D75N.

K139M and T143G cleave 2 additional targets (7CG_P and 7TT_P) as compared to D75N; however the cleavage profile of K139M and T143G is more restricted than the profile of I-*Cre*I.

These results demonstrate that the C-terminal region of I-*Cre*I is essential for HE activity. Moreover the mutations in the flanking residues of the SKTRKTT region demonstrate that they control not only nucleic acid binding, but also target specificity.

## Claims

1. A method for engineering an I-*Cre*I variant having novel substrate specificity, comprising at least the following steps:
(a) the mutation of at least one amino acid residue selected from the group consisting of residues in positions 138 and 142 by reference to I-*Cre*I amino acid sequence numbering as disclosed in SEQ ID NO: 2, Fig. 2, and
(b) the selection and/or screening of the variants from step (a) having a pattern of cleaved DNA targets that is different from that of the wild type I-*Cre*I of Fig. 8, Fig. 9, Fig. 10 and Fig. 11, or having a pattern of cleaved DNA targets that is different to a functional variant of wild type I-*Cre*I which cleaves a different starting pattern of DNA targets that is different from that of the wild type I-*Cre*I.

2. The method of claim 1, **characterized in** comprising in said variant the mutation of at least one additional residue selected in the group 139, and/or 143, by reference to I-*Cre*I amino acid sequence numbering as disclosed in SEQ ID NO:2, Fig. 2.

3. The method of claim 1 or 2, **characterized in that** the residues in positions 138 and/or 139 are substituted by an hydrophobic amino acid and/or the residues in positions 142 and/or 143 are substituted by a small amino acid, preferably the residue in position 138 is substituted by an alanine, the residue in position 139 is substituted by a methionine, and/or the residues in positions 142 and/or 143 are substituted by glycines.

4. The method of anyone of claims 1, 2 and 3, **characterized in that** step (a) comprises the mutation of two residues, each one from a different pair chosen from the residues in positions 138 and 139 and the residues in positions 142 and 143.

5. The method of anyone of claims 1 to 4, **characterized in that** said I-*Cre*I variant is an homodimer, having mutations in positions 26 to 40 and 44 to 77 of I-*Cre*I and cleaving a palindromic DNA sequence, wherein at least the nucleotides in positions + 3 to + 5 and + 8 to +10 or - 10 to - 8 and -5 to -3 of one half of said DNA sequence correspond to the nucleotides in positions + 3 to + 5 and + 8 to +10 or - 10 to - 8 and -5 to -3 of one half of a genomic DNA target from a gene of interest.

6. The method of anyone of claims 1 to 5, **characterized in that** step (a) comprises, simultaneously or subsequently, the mutation of at least one amino acid residue in a first functional subdomain corresponding to that situated from positions 26 to 40 of I-*Cre*I amino acid sequence, that alter the specificity towards the nucleotide in positions ± 8 to 10 of the DNA target, and/or the mutation of at least amino acid residue in a second functional subdomain corresponding to that situated from positions 44 to 77 of I-*Cre*I amino acid sequence, that alter the specificity towards the nucleotide in positions ± 3 to 5 of the DNA target, preferably step (a) further comprises, simultaneously or subsequently, the random mutation of the whole or the C-terminal half of said I-*Cre*I variant amino acid sequence.

7. The method of anyone of claims 1 to 6, **characterized in that** step (b) comprises the selection and/or screening of the variants from step (a) which are able to cleave at least one DNA target sequence that is not cleaved by said parent I-*Cre*I, said DNA target sequence being derived from the parent I-*Cre*I cleavage site, by the replacement of at least one nucleotide of one half of said cleavage site, with a different nucleotide, said DNA target sequence is derived from the I-*Cre*I palindromic site having the sequence SEQ ID NO: 1, preferably said DNA target has mutation(s) in the nucleotide(s) in positions ± 1 to 2, ± 6 to 7, ± 8 to 10 and/or ± 11 to 12, in particular said DNA target sequence is a genomic sequence which is present in a gene of interest.

8. An homodimeric or heterodimeric I-*Cre*I variant comprising at least one mutation in position 138 and/or 142 and having a pattern of cleaved DNA targets that is different from that of the parent I-*Cre*I, **characterized in that** it is obtainable by the method of anyone of claims 1 to 7, with the exclusion of the homodimeric variants of SEQ ID NO: 3 and 4 and the homo- and hetero-dimeric variants comprising a monomer of SEQ ID NO: 5.

9. The variant of claim 8, **characterized in that** it is an heterodimer comprising the monomers of two different variants obtainable by the method of any one of claims 1 to 7.

10. The variant of claim 8 or claim 9, **characterized in that** it is an I-*Cre*I variant having one or two mutations, each one from a different pair of mutations selected from the group consisting of the pair S138A and K139M and the pair K142G and T143G, preferably said variant is of the sequence SEQ ID NO: 6 to 9.

11. The variant of claim 10, which is an heterodimeric I-*Cre*I variant consisting of two monomers, each monomer further comprising different mutations in positions 26 to 40 and 44 to 77 of I-*Cre*I, said variant being able to cleave a genomic DNA target from a gene of interest.

12. A single-chain chimeric meganuclease comprising two monomers or core domains of one or two variants of anyone of claims 8 to 11, or a combination of both.

13. A polynucleotide fragment encoding one monomer of the variant of anyone of claims 8 to 11 or the single-chain meganuclease of claim 12.

14. A recombinant vector, **characterized in that** it comprises at least one polynucleotide fragment of claim 13, preferably two polynucleotide fragments each encoding one of the two monomers of an heterodimeric variant of anyone of claims 8 to 11, said fragment(s) being operatively linked to regulatory sequences allowing the production of the two monomers or a polynucleotide fragment encoding the single-chain meganuclease of claim 12, said fragment being operatively linked to regulatory sequences allowing the production of said single-chain meganuclease, said vector further including preferably a targeting DNA construct comprising sequences sharing homologies with the region surrounding the genomic DNA target sequence as defined in anyone of claims 5, 7 and 11, said targeting DNA construct comprising preferably: a) sequences sharing homologies with the region surrounding the genomic DNA target sequence as defined in anyone of claims 5, 7 and 11, and b) sequences to be introduced flanked by sequence as in a).

15. A non-human transgenic animal, a transgenic plant or a host cell comprising one or two polynucleotide fragments as defined in claim 13 or claim 14.

16. A pharmaceutical composition, **characterized in that** it comprises at least a variant of anyone of claims 8 to 11, a single-chain meganuclease of claim 12, one or two polynucleotide fragments as defined in claim 13 or claim 14, a vector of claim14, preferably it further comprises a targeting DNA construct comprising the sequence which repairs the genomic site of interest flanked by sequences sharing homologies with the targeted locus.

17. A method for decreasing the toxicity of a parent I-*Cre*I, comprising: the mutation of at least one amino acid of the final C-terminal loop selected from the group 138, 142 of said parent I-*Cre*I.

## Patentansprüche

1. Verfahren zum Entwickeln einer I-*Cre*I-Variante, die eine neue Substratspezifität aufweist, das mindestens die folgenden Schritte aufweist:
(a) die Mutation mindestens eines Aminosäurerestes, der aus der Gruppe ausgewählt ist, die aus den Resten in den Positionen 138 und 142 durch Bezugnahme auf die I-*Cre*I Aminosäuresequenznummerierung, wie in SEQ ID NO:2, Fig. 2 offenbart, besteht, und
(b) die Auswahl und/oder das Screening der Varianten aus Schritt (a), die ein Muster an gespaltenen DNS-Targets aufweisen, das sich von dem der Wildtyp *I-CreI* aus Fig. 8, Fig. 9, Fig. 10 und Fig. 11 unterscheidet, oder die ein Muster gespaltener DNS-Targets aufweisen, das sich von dem einer funktionellen Variante von Wildtyp I-*Cre*I unterscheidet, die ein unterschiedliches Startmuster an DNS-Targets spaltet, das sich von dem der Wildtyp I-*Cre*I unterscheidet.

2. Verfahren nach Anspruch 1, **dadurch gekennzeichnet, dass** es in der beschriebenen Variante die Mutation mindestens eines zusätzlichen Restes aufweist, der in der Gruppe 139, und/oder 143 durch Bezugnahme auf I-*Cre*I-Aminosäuresequenznummerierung, wie in SEQ ID NO:2, Fig.2 offenbart, ausgewählt ist.

3. Verfahren nach Anspruch 1 oder 2, **dadurch gekennzeichnet, dass** die Reste in Positionen 138 und/oder 139 durch eine hydrophobe Aminosäure ersetzt sind und/oder die Reste in Positionen 142 und/oder 143 durch eine kleine Aminosäure ersetzt sind, bevorzugt der Rest in Position 138 durch ein Alanin ersetzt ist, der Rest in Position 139 durch ein Methionin ersetzt ist, und/oder die Reste in Positionen 142 und/oder 143 durch Glycin ersetzt sind.

4. Verfahren nach einem der Ansprüche 1, 2 und 3, **dadurch gekennzeichnet, dass** Schritt (a) die Mutation zweier Reste aufweist, wobei jeder von einem unterschiedlichen Paar ist, das von den Resten in Positionen 138 und 139 und den Resten in Positionen 142 und 143 ausgewählt ist.

5. Verfahren nach einem der Ansprüche 1 bis 4, **dadurch gekennzeichnet, dass** die beschriebene I-*Cre*I-Variante ein Homodimer ist, das Mutationen in den Positionen 26 bis 40 und 44 bis 77 von I-*Cre*I aufweist, und das eine palindromische DNS-Sequenz spaltet, wobei mindestens die Nukleotide in Positionen + 3 bis + 5 und + 8 bis + 10 oder - 10 bis - 8 und - 5 bis - 3 von einer Hälfte der beschriebenen DNS-Sequenz den Nukleotiden in Positionen + 3 bis + 5 und + 8 bis + 10 oder - 10 bis - 8 und - 5 bis - 3 von einer Hälfte eines genomischen DNS-Targets von einem Gen von Interesse entsprechen.

6. Verfahren nach einem der Ansprüche 1 bis 5, **dadurch gekennzeichnet, dass** Schritt (a) simultan oder nacheinander folgend die Mutation von mindestens einem Aminosäurerest in einer ersten funktionalen Unterdomäne umfasst, aufweist, die der entspricht, die von Positionen 26 bis 40 der I-*Cre*I-Aminosäuresequenz befindlich ist, welche die Spezifität in Richtung des Nukleotides in Positionen ± 8 bis 10 des DNS-Targets verändern, und/oder die Mutation mindestens eines Aminosäurerestes in einer zweiten funktionalen Subdomäne, die der entspricht, die von Positionen 44 bis 77 der I-*Cre*I-Aminosäuresequenz befindlich ist, welche die Spezifität in Richtung des Nukleotides in Positionen ± 3 bis 5 des DNS-Targets verändern, Schritt (a) bevorzugt weiterhin simultan oder nacheinander folgend die zufällige Mutation der gesamten oder der C-terminalen Hälfte der beschriebenen I-*Cre*I-Varianten Aminosäuresequenz aufweist.

7. Verfahren nach einem der Ansprüche 1 bis 6, **dadurch gekennzeichnet, dass** Schritt (b) die Auswahl und/oder das Screening von den Varianten aus Schritt (a) umfasst, die in der Lage sind, mindestens eine DNS-Targetsequenz zu spalten, die nicht von der beschriebenen Ursprungs-I-*Cre*I gespalten wird, wobei die beschriebene DNS-Targetsequenz durch den Austausch mindestens eines Nukleotides von einer Hälfte von der beschriebenen Schnittstelle mit einem anderen Nukleotid von der ursprünglichen I-*Cre*I-Schnittstelle abgeleitet ist, wobei die beschriebene DNS-Targetsequenz von der palindromischen I-*Cre*I-Stelle abgeleitet ist, welche die Sequenz SEQ ID NO: 1 aufweist, wobei das beschriebene DNS-Target bevorzugt Mutation(en) in dem(den) Nukleotid(en) in Positionen ± 1 bis 2, ± 6 bis 7, ± 8 bis 10 und/oder ± 11 bis 12 aufweist, wobei insbesondere die beschriebene DNS-Targetsequenz eine genomische Sequenz ist, die in einem Gen von Interesse vorhanden ist.

8. Homodimere oder heterodimere I-*Cre*I-Variante, die mindestens eine Mutation in Position 138 und/oder 142 aufweist und, die ein Muster geschnittener DNS-Targets aufweist, das sich von dem der ursprünglichen I-*Cre*I unterscheidet, **dadurch gekennzeichnet, dass** sie durch das Verfahren nach einem der Ansprüche 1 bis 7 erhältlich ist, mit der Ausnahme der homodimeren Varianten nach SEQ ID NO:3 und 4 und der homo- und heterodimeren Varianten, die ein Monomer nach SEQ ID NO: 5 umfassen.

9. Variante nach Anspruch 8, **dadurch gekennzeichnet, dass** sie ein Heterodimer ist, das die Monomere von zwei unterschiedlichen Varianten aufweist, die durch die Verfahren nach einem der Ansprüche 1 bis 7 erhältlich sind.

10. Variante nach Anspruch 8 oder Anspruch 9, **dadurch gekennzeichnet, dass** sie eine I-*Cre*I-Variante ist, die eine oder zwei Mutationen aufweist, wobei jede von einem unterschiedlichen Paar an Mutationen ist, die aus der Gruppe ausgewählt sind, die aus dem Paar S138A und K139M und dem Paar K142G und T143G besteht, wobei die beschriebene Variante bevorzugt nach der Sequenz SEQ ID NO: 6 bis 9 ist.

11. Variante nach Anspruch 10, die eine heterodimere I-*Cre*I-Variante ist, die aus zwei Monomeren besteht, wobei jedes Monomer weiterhin unterschiedliche Mutationen in Positionen 26 bis 40 und 44 bis 77 der I-*Cre*I aufweist, wobei die beschriebene Variante in der Lage ist, ein genomisches DNS-Target von einem Gen von Interesse zu spalten.

12. Chimere Single-Chain-Meganuklease, die zwei Monomere oder Kemdomänen von ein oder zwei Varianten nach einem der Ansprüche 8 bis 11 oder eine Kombination von beiden aufweist.

13. Polynukleotidfragment, das für ein Momomer der Variante nach einem der Ansprüche 8 bis 11 oder der Single-Chain-Meganuklease nach Anspruch 12 kodiert.

14. Rekombinanter Vektor, **dadurch gekennzeichnet, dass** er mindestens ein Polynukleotidfragment nach Anspruch 13 umfasst, bevorzugt zwei Polynukleotidfragemente, wobei jedes für eines der zwei Monomere einer heterodimeren Variante nach einem der Ansprüche 8 bis 11 kodiert, wobei das/die beschriebene(n) Fragment(e) operativ mit regulatorischen Sequenzen zusammenhängen, was die Produktion der zwei Monomere erlaubt, oder ein Polynukleotidfragment, das für die Single-Chain-Meganuklease nach Anspruch 12 kodiert, wobei das beschriebene Fragment operativ mit regulatorischen Sequenzen zusammenhängt, was die Produktion der beschriebenen Single-Chain-Meganuklease erlaubt, wobei der beschriebene Vektor weiterhin bevorzugt ein Target-DNS-Konstrukt umfasst, das Sequenzen umfasst, die Homologien mit der Region teilen, welche die genomische DNS-Targetsequenz, wie in einem der Ansprüche 5, 7 und 11 definiert, umgeben, wobei das beschriebene Target-DNS-Konstrukt bevorzugt umfasst: a) Sequenzen, die Homologien mit der Region teilen, welche die genomische DNS-Targetsequenz, wie in einem der Ansprüche 5, 7 und 11 definiert, umgeben, die flankiert von einer Sequenz wie in a) einzufügen sind.

15. Nicht-humanes transgenes Tier, transgene Pflanze oder Wirtszelle, das/die eine oder zwei Polynukleotidfragmente, wie in Anpruch 13 oder Anspruch 14 definiert, umfasst.

16. Pharmazeutische Zusammensetzung, **dadurch gekennzeichnet, dass** sie mindestens eine Variante nach einem der Ansprüche 8 bis 11 umfasst, eine Single-Chain-Meganuklease nach Anspruch 12, ein oder zwei Polynukleotidfragmente, wie in Anspruch 13 oder Anspruch 14 definiert, einen Vektor nach Anspruch 14, bevorzugt umfasst sie weiterhin ein Target-DNS-Konstrukt, das die Sequenz umfasst, welche die genomische Stelle von Interesse repariert, die von Sequenzen flankiert ist, die Homologien mit dem Targetlokus teilen.

17. Verfahren zum Vermindern der Toxizität einer ursprünglichen I-*Cre*I umfassend: die Mutation mindestens einer Aminosäure der endgültigen C-terminalen Schleife, ausgewählt aus der Gruppe 138, 142 der beschriebenen ursprünglichen *I-CreI.*

## Revendications

1. Procédé pour modifier génétiquement un variant d'I-*Cre*I ayant une nouvelle spécificité de substrat, comprenant au moins les étapes suivantes :
(a) la mutation d'au moins un résidu d'acide aminé choisi dans le groupe consistant en les résidus en positions 138 et 142 par référence à la numérotation de la séquence d'acides aminés d'I-*Cre*I telle que présentée dans SEQ ID NO:2, Fig. 2, et
(b) la sélection et/ou le criblage des variants de l'étape (a) ayant un modèle de cibles d'ADN clivées qui est différent de celui d'I-CreI de type sauvage de la Fig. 8, de la Fig. 9, de la Fig. 10 et de la Fig. 11, ou ayant un modèle de cibles d'ADN clivées qui est différent de celui d'un variant fonctionnel d'I-*Cre*I de type sauvage, qui clive un modèle de départ différent de cibles d'ADN, qui est différent de celui de l'I-*Cre*I de type sauvage.

2. Procédé de la revendication 1, **caractérisé en ce qu'**il comprend, dans ledit variant, la mutation d'au moins un résidu additionnel choisi dans le groupe 139 et/ou dans le groupe 143, par référence à la numérotation de la séquence d'acides aminés d'I-*Cre*I telle que présentée dans SEQ ID NO:2, Fig. 2.

3. Procédé de la revendication 1 ou 2, **caractérisé en ce que** les résidus en positions 138 et/ou 139 sont remplacés par un acide aminé hydrophobe, et/ou les résidus en positions 142 et/ou 143 sont remplacés par un petit acide aminé, de préférence le résidu en position 138 est remplacé par une alanine, le résidu en position 139 est remplacé par une méthionine, et/ou les résidus en positions 142 et/ou 143 sont remplacés par des glycines.

4. Procédé selon l'une quelconque des revendications 1, 2 et 3, **caractérisé en ce que** l'étape (a) comprend la mutation de deux résidus, chacun provenant d'une paire différente choisie parmi les résidus en positions 138 et 139 et les résidus en positions 142 et 143.

5. Procédé selon l'une quelconque des revendications 1 à 4, **caractérisé en ce que** ledit variant d'I-CreI est un homodimère, ayant des mutations en positions 26 à 40 et 44 à 77 d'I-*Cre*I et clivant une séquence d'ADN palindromique, les nucléotides en position +3 à +5 et +8 à +10, ou -10 à -8 et -5 à -3, d'une moitié de ladite séquence d'ADN, correspondant aux nucléotides en positions +3 à +5 et +8 à +10, ou -10 à -8 et -5 à -3, d'une moitié d'une cible d'ADN génomique provenant d'un gène d'intérêt.

6. Procédé selon l'une quelconque des revendications 1 à 5, **caractérisé en ce que** l'étape (a) comprend, simultanément ou successivement, la mutation d'au moins un résidu d'acide aminé dans un premier sous-domaine fonctionnel correspondant à celui qui est situé de la position 26 à la position 40 de la séquence d'acides aminés d'I-*Cre*I, qui altèrent la spécificité vis-à-vis du nucléotide en positions ± 8 à 10 de la cible d'ADN, et/ou la mutation d'au moins un résidu d'acide aminé dans un deuxième sous-domaine fonctionnel correspondant à celui qui est situé de la position 44 à la position 77 de la séquence d'acides aminés d'I-*Cre*I, qui altèrent la spécificité vis-à-vis du nucléotide en positions ± 3 à 5 de la cible d'ADN, l'étape (a) comprenant en outre de préférence, simultanément ou successivement, la mutation aléatoire de la totalité ou de la moitié C-terminale de ladite séquence d'acide aminé du variant d'I-*Cre*I.

7. Procédé selon l'une quelconque des revendications 1 à 6, **caractérisé en ce que** l'étape (b) comprend la sélection et/ou le criblage des variants de l'étape (a) qui sont à même de cliver au moins une séquence cible d'ADN qui n'est pas clivée par ladite I-*Cre*I parente, ladite séquence cible d'ADN dérivant du site de clivage par l'I-*Cre*I parente, par remplacement d'au moins un nucléotide d'une moitié dudit site de clivage par un nucléotide différent, ladite séquence cible d'ADN dérivant du site palindromique d'I-*Cre*I ayant la séquence SEQ ID NO:1, de préférence ladite cible d'ADN ayant une ou plusieurs mutations dans le ou les nucléotides en positions ± 1 à 2, ± 6 à 7, ± 8 à 10 et/ou ± 11 à 12, en particulier ladite séquence cible d'ADN étant une séquence génomique qui est présente dans un gène d'intérêt.

8. Variant d'I-*Cre*I homodimère ou hétérodimère comprenant au moins une mutation en positions 138 et/ou 142 et ayant un profil de cibles d'ADN clivées qui est différent de celui de l'I-*Cre*I parente, **caractérisé en ce qu'**il peut être obtenu par le procédé selon l'une quelconque des revendications 1 à 7, à l'exclusion des variants homodimères SEQ ID NO:3 et 4 et des variants homo- et hétérodimères comprenant un monomère SEQ ID NO:5.

9. Variant selon la revendication 8, **caractérisé en ce qu'**il s'agit d'un hétérodimère comprenant les monomères de deux variants différents pouvant être obtenus par le procédé selon l'une quelconque des revendications 1 à 7.

10. Variant selon la revendication 8 ou la revendication 9, **caractérisé en ce qu'**il s'agit d'un variant d'I-*Cre*I ayant une ou deux mutations, chacune provenant d'une paire différente de mutations choisies dans le groupe consistant en les paires S138A et K139M et les paires K142G et T143G, de préférence ledit variant ayant la séquence SEQ ID NO:6 à 9.

11. Variant selon la revendication 10, qui est un variant d'I-*Cre*I hétérodimère consistant en deux monomères, chaque monomère comprenant en outre des mutations différentes en positions 26 à 40 et 44 à 77 d'I-*Cre*I, ledit variant étant à même de cliver une cible d'ADN génomique provenant d'un gène d'intérêt.

12. Méganucléase chimère monocaténaire comprenant deux monomères ou domaines centraux de l'un des variants, ou des deux, de l'une quelconque des revendications 8 à 11, ou d'une combinaison des deux.

13. Fragment polynucléotidique codant pour un monomère du variant selon l'une quelconque des revendications 8 à 11 ou de la méganucléase monocaténaire de la revendication 12.

14. Vecteur recombinant, **caractérisé en ce qu'**il comprend au moins un fragment polynucléotidique selon la revendication 13, de préférence deux fragments polynucléotidiques dont chacun code pour l'un des deux monomères d'un variant hétérodimère selon l'une quelconque des revendications 8 à 11, ledit ou lesdits fragments étant fonctionnellement liés à des séquences régulatrices permettant la production des deux monomères, ou un fragment polynucléotidique codant pour la méganucléase monocaténaire selon la revendication 12, ledit fragment étant fonctionnellement lié à des séquences régulatrices permettant la production de ladite méganucléase monocaténaire, ledit vecteur comprenant en outre de préférence une construction d'ADN de ciblage comprenant des séquences partageant une homologie avec la région entourant la séquence cible d'ADN génomique telle que définie dans l'une quelconque des revendications 5, 7 et 11, ladite construction d'ADN de ciblage comprenant de préférence : a) des séquences partageant une homologie avec la région entourant la séquence cible d'ADN génomique telle que définie dans l'une quelconque des revendications 5, 7 et 11, et b) des séquences destinées à être introduites en étant flanquées d'une séquence telle qu'en a).

15. Animal transgénique non humain, plante transgénique ou cellule hôte comprenant un ou deux fragments polynucléotidiques tels que définis dans la revendication 13 ou la revendication 14.

16. Composition pharmaceutique, **caractérisée en ce qu'**elle comprend au moins un variant selon l'une quelconque des revendications 8 à 11, une méganucléase monocaténaire selon la revendication 12, un ou deux fragments polynucléotidiques tels que définis dans la revendication 13 ou la revendication 14, un vecteur selon la revendication 14, et de préférence comprend en outre une construction d'ADN de ciblage comprenant la séquence qui répare le site génomique d'intérêt flanqué de séquences partageant une homologie avec le locus ciblé.

17. Procédé pour diminuer la toxicité d'une I-CreI parente, comprenant la mutation d'au moins un acide aminé de la boucle C-terminale finale, choisi dans le groupe 138, 142 de l'I-*Cre*I parente.


### SEQUENCE LISTING

<110> CELLECTIS MONTOYA, Guillermo BLANCO, Francisco PRIETO, Jesus
<120> LAGLIDADG HOMING ENDONUCLESAE VARIANTS HAVING NOVEL SUBSTRATE SPECIFICITY AND USE THEREOF
<130> 1546pct15
<160> 31
<170> PatentIn version 3.3
<210> 1
   <211> 24
   <212> DNA
   <213> artificial sequence
<220>
   <223> C1221 target
<400> 1
   tcaaaacgtc gtacgacgtt ttga 24
<210> 2
   <211> 163
   <212> PRT
   <213> chlamydomonas reinhardtii
<400> 2
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Tyr Lys Phe Lys His Gln Leu Ser Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 3
   <211> 163
   <212> PRT
   <213> artificial sequence
<220>
   <223> I-CreI D137E
<400> 3
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Glu Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 4
   <211> 163
   <212> PRT
   <213> artificial sequence
<220>
   <223> I-CreI K142Q
<400> 4
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Gln Thr Thr
   130 135 140
   Ser Glu Thr Val Arg Ala Val Leu Asp Ser Leu Ser Glu Lys Lys Lys
   145 150 155 160
   Ser Ser Pro
<210> 5
   <211> 167
   <212> PRT
   <213> artificial sequence
<220>
   <223> I-Cre insA2, S32T, Y33H, A42T, Q44K, R68Y, R70S, I77R, Q92R, K96R, K107R, W110E, R111Q, I132V, T140A, T143A, insAAD 164-166
<400> 5
   Met Ala Asn Thr Lys Tyr Asn Lys Glu Phe Leu Leu Tyr Leu Ala Gly
   1 5 10 15
   Phe Val Asp Gly Asp Gly Ser Ile Ile Ala Gln Ile Lys Pro Asn Gln
   20 25 30
   Thr His Lys Phe Lys His Gln Leu Ser Leu Thr Phe Lys Val Thr Gln
           35                  40                  45
   Lys Thr Gln Arg Arg Trp Phe Leu Asp Lys Leu Val Asp Glu Ile Gly
       50                  55                  60
   Val Gly Tyr Val Tyr Asp Ser Gly Ser Val Ser Asp Tyr Arg Leu Ser
   65                  70                  75                  80
   Glu Ile Lys Pro Leu His Asn Phe Leu Thr Gln Leu Arg Pro Phe Leu
                   85                  90                  95
   Arg Leu Lys Gln Lys Gln Ala Asn Leu Val Leu Arg Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Val Ala Ala Leu Asn Asp Ser Lys Ala Arg Lys Ala
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Asp
                   165
<210> 6
   <211> 163
   <212> PRT
   <213> artificial sequence
<220>
   <223> I-CreI S138A
<400> 6
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Tyr Lys Phe Lys His Gln Leu Ser Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ala Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 7
   <211> 163
   <212> PRT
   <213> artificial sequence
<220>
   <223> I-CreI K139M
<400> 7
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Met Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 8
   <211> 163
   <212> PRT
   <213> artificial sequence
<220>
   <223> I-CreI K142G
<400> 8
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Gly Thr Thr
   130 135 140
   Ser Glu Thr Val Arg Ala Val Leu Asp Ser Leu Ser Glu Lys Lys Lys
   145 150 155 160
   Ser Ser Pro
<210> 9
   <211> 163
   <212> PRT
   <213> artificial sequence
<220>
   <223> I-CreI T143G
<400> 9
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Tyr Lys Phe Lys His Gln Leu Ser Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Gly Thr
       130                 135                 140
   Ser Glu Thr Val Arg Ala Val Leu Asp Ser Leu Ser Glu Lys Lys Lys
   145                 150                 155                 160
   Ser Ser Pro
<210> 10
   <211> 24
   <212> DNA
   <213> artificial sequence
<220>
   <223> 10 GGG_P target
<400> 10
   tcgggacgtc gtacgacgtc ccga 24
<210> 11
   <211> 24
   <212> DNA
   <213> artificial sequence
<220>
   <223> 10 GGA_P target
<400> 11
   tcggaacgtc gtacgacgtt ccga 24
<210> 12
   <211> 24
   <212> DNA
   <213> artificial sequence
<220>
   <223> 10 GGT_P target
<400> 12
   tcggtacgtc gtacgacgta ccga 24
<210> 13
   <211> 24
   <212> DNA
   <213> artificial sequence
<220>
   <223> 10 GGC_P target
<400> 13
   tcggcacgtc gtacgacgtg ccga 24
<210> 14
   <211> 24
   <212> DNA
   <213> artificial sequence
<220>
   <223> 10 GAG_P target
<400> 14
   tcgagacgtc gtacgacgtc tcga 24
<210> 15
   <211> 24
   <212> DNA
   <213> artificial target
<220>
   <223> 10 GAA_P target
<400> 15
   tcgaaacgtc gtacgacgtt tcga 24
<210> 16
   <211> 24
   <212> DNA
   <213> artificial sequence
<220>
   <223> 10 GAT_P target
<400> 16
   tcgatacgtc gtacgacgta tcga 24
<210> 17
   <211> 24
   <212> DNA
   <213> artificial sequence
<220>
   <223> I-CreI target
<400> 17
   tcaaaacgtc gtgagacagt ttgg 24
<210> 18
   <211> 29
   <212> DNA
   <213> artificial sequence
<220>
   <223> primer
<400> 18
   gatataccat ggccaatacc aaatataac 29
<210> 19
   <211> 47
   <212> DNA
   <213> artificial sequence
<220>
   <223> primer
<400> 19
   ttatcagtcg gccgcatcgt tcagagctgc aatctgatcc acccagg 47
<210> 20
   <211> 37
   <212> DNA
   <213> artificial sequence
<220>
   <223> primer
<400> 20
   gagtgcggcc gcagtggttt tacgcgtctt agaatcg 37
<210> 21
   <211> 24
   <212> DNA
   <213> artificial sequence
<220>
   <223> DNA target
<400> 21
   gcaaaacgtc gtgagacagt ttgc 24
<210> 22
   <211> 44
   <212> PRT
   <213> artificial sequence
<220>
   <223> I-CreI C-term sequence 120-
<400> 22
   Asp Lys Phe Leu Glu Val Cys Thr Trp Val Asp Gln Ile Ala Ala Leu
   1 5 10 15
   Asn Asp Ser Lys Thr Arg Lys Thr Thr Ser Glu Thr Val Arg Ala Val
   20 25 30
   Leu Asp Ser Leu Ser Glu Lys Lys Lys Ser Ser Pro
   35 40
<210> 23
   <211> 44
   <212> PRT
   <213> artificial sequence
<220>
   <223> Tmu2593c C-term sequence 123-
<400> 23
   Gln Ala Phe Phe Lys Leu Cys Glu Gln Val Asp Gln Phe Ser Glu Phe
   1 5 10 15
   Asn Asp Ser Lys Lys Arg Lys Leu Thr Ser Gln Val Val Arg Ser Glu
   20 25 30
   Ile Gly Thr Ala Ile His Leu Phe Pro Val Glu Thr
   35 40
<210> 24
   <211> 44
   <212> PRT
   <213> artificial sequence
<220>
   <223> I-PakI C-term sequence 123-
<400> 24
   Gln Leu Phe Leu Lys Leu Cys Glu Gln Val Asp His Phe Ser Glu Phe
   1 5 10 15
   Asn Asp Ser Lys Lys Arg Lys Leu Thr Ser Gln Val Val Arg Ser Glu
   20 25 30
   Ile Gly Glu Thr Leu Asn Leu Phe Pro Val Glu Thr
   35 40
<210> 25
   <211> 44
   <212> PRT
   <213> artificial sequence
<220>
   <223> sdu2593c C-term sequence 122-
<400> 25
   Gln Ser Phe Val Lys Leu Cys Glu Arg Val Asp Ser Ile Ala Lys Leu
   1               5                   10                  15
   Asn Asp Ser Lys Lys Arg Ile Ile Thr Ser Asp Val Val Arg Ser Ile
               20                  25                  30
   Leu Leu Asn Ser Val Gln Lys Phe Pro Val Glu Thr
           35                  40
<210> 26
   <211> 39
   <212> PRT
   <213> artificial sequence
<220>
   <223> Sob2593c C-term sequence 129-
<400> 26
   Val Leu Phe Val Gln Leu Cys Arg Leu Thr Asp Gln Val Ala Leu Leu
   1               5                   10                  15
   Asn Asp Ser Lys Ser Arg Thr Ile Thr Ala Glu Val Val Glu Thr Thr
               20                  25                  30
   Leu Arg Glu Leu Gly Leu Ile
           35
<210> 27
   <211> 44
   <212> PRT
   <213> artificial sequence
<220>
   <223> Clu2593c C-term sequence 119-
<400> 27
   Glu Ile Phe Leu Glu Leu Cys Asn Val Val Asp His Val Ala Thr Leu
   1               5                   10                  15
   Asn Asp Thr Lys Lys Arg Lys Tyr Thr Ala Glu Ile Val Ala Ala Lys
               20                  25                  30
   Leu Lys Glu Leu Lys Glu Cys Val Val Pro Val Glu
           35                  40
<210> 28
   <211> 40
   <212> PRT
   <213> artificial sequence
<220>
   <223> I-CvuI C-term sequence 122-
<400> 28
   Ser Val Leu Met Glu Ala Ala Leu Leu Ala Asp Lys Val Gly Leu Leu
   1               5                   10                  15
   Thr Asp Gly Lys Lys Arg Thr Ile Leu Ala Glu Asn Val Arg Glu Cys
               20                  25                  30
   Leu Lys Lys Leu Gly His Val Val
           35                  40
<210> 29
   <211> 43
   <212> PRT
   <213> artificial sequence
<220>
   <223> Mvi2593m c-term sequence 120-
<400> 29
   Ala Asp Phe Ile Glu Val Cys Gln Leu Val Asp Lys Ile Ala Glu Gln
   1               5                   10                  15
   Asn Phe Ser Lys Lys Arg Ile Ile Thr Ser Lys Val Val Glu Ala Thr
               20                  25                  30
   Leu Met Leu Pro Val Glu Thr Glu Lys Ser Ser
           35                  40
<210> 30
   <211> 41
   <212> PRT
   <213> artificial sequence
<220>
   <223> Aca2593m C-term sequence 124-
<400> 30
   Ala Gly Phe Leu Glu Val Cys Lys Leu Val Asp Glu Val Ala Asp His
   1 5 10 15
   Thr Tyr Ser Lys Lys Arg Lys Asn Thr Ser Leu Thr Val Lys Asn Ser
   20 25 30
   Leu Leu Leu Pro Val Glu Thr Glu Glu
   35 40
<210> 31
   <211> 41
   <212> PRT
   <213> artificial sequence
<220>
   <223> Nol2593m c-term sequence 123-
<400> 31
   Ala Gly Phe Leu Glu Val Cys Lys Leu Val Asp Glu Val Ala Asp His
   1 5 10 15
   Thr Tyr Ser Lys Lys Arg Lys Asn Thr Ser Leu Thr Val Lys Asn Ser
   20 25 30
   Leu Leu Leu Pro Val Glu Thr Glu Glu
   35 40